(19) Europäisches Patentamt
European Patent Office
Office européen des brevets

(11) EP 4 725 968 A1

(12) EUROPEAN PATENT APPLICATION
published in accordance with Art. 153(4) EPC

(43) Date of publication:
**15.04.2026 Bulletin 2026/16**

(21) Application number: **24822763.9**

(22) Date of filing: **14.06.2024**

(51) International Patent Classification (IPC):
***C07K 16/30*** (2006.01) ***C07K 16/18*** (2006.01)
***C12N 15/13*** (2006.01) ***A61P 35/00*** (2006.01)
***A61K 39/00*** (2006.01) ***A61K 39/395*** (2006.01)
***A61K 47/68*** (2017.01)

(52) Cooperative Patent Classification (CPC):
**A61K 39/00; A61K 39/395; A61K 47/68; A61P 35/00; C07K 16/00; C07K 16/18; C07K 16/30**

(86) International application number:
**PCT/CN2024/099094**

(87) International publication number:
**WO 2024/255815 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.06.2023 CN 202310720746**
**01.09.2023 CN 202311126022**
**18.09.2023 CN 202311211948**

(71) Applicant: **Salubris (Chengdu) Biotech Co., Ltd.**
**Chengdu, Sichuan 610041 (CN)**

(72) Inventors:
- **MOU, Zongchun**
  **Chengdu, Sichuan 610041 (CN)**
- **LI, Ning**
  **Chengdu, Sichuan 610041 (CN)**
- **LIU, Xiaohong**
  **Chengdu, Sichuan 610041 (CN)**
- **XIA, Xiaozhen**
  **Chengdu, Sichuan 610041 (CN)**
- **PENG, Xiaolun**
  **Chengdu, Sichuan 610041 (CN)**
- **XIONG, Xiaolin**
  **Shenzhen, Guangdong 518017 (CN)**

(74) Representative: **Groth & Co. KB**
**P.O. Box 6107**
**102 32 Stockholm (SE)**

Remarks:
The complete document including Reference Table(s) and the Sequence Listing(s) can be downloaded from the EPO website

# (54) ANTI-GPC3 ANTIBODY OR ANTIGEN-BINDING FRAGMENT AND USE THEREOF

(57) Provided in the present invention are an anti-GPC-3 antibody and an antigen-binding fragment thereof, and also provided are a polynucleotide encoding the antibody, an expression vector and a host cell for expressing the antibody, and a method for preparing the antibody. In addition, further provided in the present invention are a pharmaceutical composition comprising the antibody and the use of the antibody in the preparation of a drug for treating cancers.

EP 4 725 968 A1

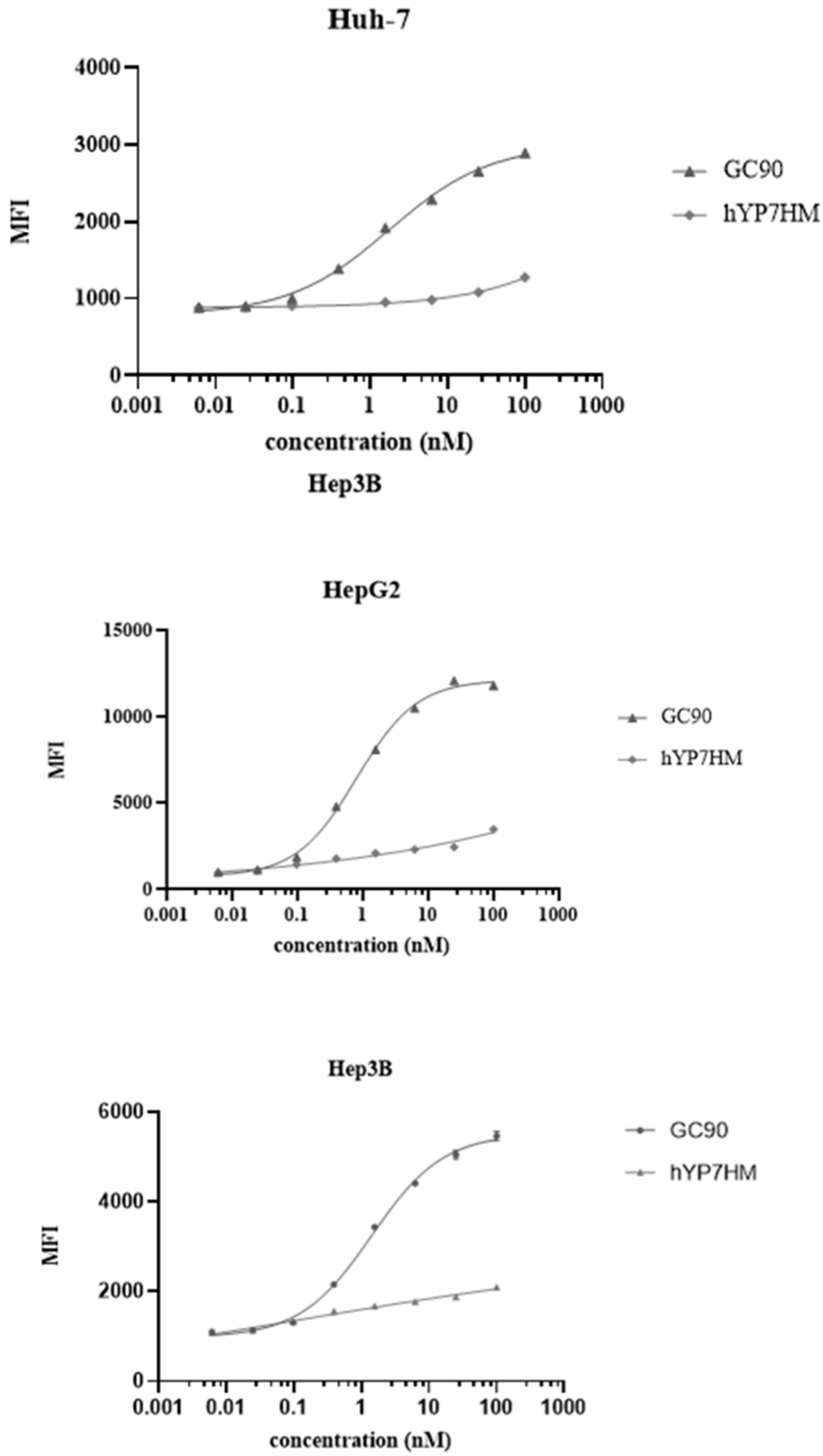
Huh-7
4000
3000
2000
1000
0
MFI
0.001
0.01
0.1
1
10
100
1000
concentration (nM)
GC90
hYP7HM
Hep3B
HepG2
15000
10000
5000
0
MFI
0.001
0.01
0.1
1
10
100
1000
concentration (nM)
GC90
hYP7HM
Hep3B
6000
4000
2000
0
MFI
0.001
0.01
0.1
1
10
100
1000
concentration (nM)
GC90
hYP7HM

FIG. 4

## Description

### TECHNICAL FIELD

The present invention belongs to the field of biomedical technology, and in particular to an anti-GPC3 antibody or an antigen-binding fragment and use thereof.

### BACKGROUND

Glypican-3 (GPC3) is a heparan sulfate proteoglycan expressed on the surface of various malignant cells, such as hepatocellular carcinoma (HCC) cells. GPC3 is linked to the cell surface via a glycosylphosphatidylinositol (GPI) anchor. GPC3 has been shown to be highly expressed in more than 70% of HCC biopsy tissues, but not in adjacent non-tumor tissues. Patients with GPC3-positive HCC have significantly lower disease-free survival rates than those with GPC3-negative HCC.

Antibodies binding to GPC3 have been identified to exhibit cell growth-inhibitory effects through antibody-dependent cytotoxicity (ADCC) or complement-dependent cytotoxicity (CDC) activities (International Patent Application WO 2003/000883). Moreover, GPC3 has been shown to undergo cleavage *in vivo* and to be secreted into the bloodstream in a soluble form, and antibodies capable of detecting the secreted form of GPC3 can be utilized for tumor diagnosis (International Patent Applications WO 2004/022739, WO 03/100429, and WO 2004/018667).

Antibody-drug conjugates (ADCs) are a class of novel targeted therapeutic agents, in which an antibody is conjugated to a highly cytotoxic small-molecule drug. They combine the potent cell-killing ability of small molecules with the high targeting specificity of monoclonal antibodies, making them the focus of research and development in tumor-targeted therapy. Typically, an ADC includes three main components linked in a defined manner: an antibody or antibody-like ligand, a linker, and a small-molecule drug. The targeting specificity of an ADC derives from the antibody component, while its cytotoxicity primarily comes from the small-molecule drug. The antibody component itself may also contribute to cytotoxic effects. Upon binding to a tumor cell surface antigen, the antibody component is endocytosed into the cell. Subsequently, the ADC is broken down in lysosomes, releasing the active cytotoxic payload, which disrupts DNA or inhibits tumor cell division, ultimately killing the tumor cells. Compared with other therapeutic modalities, ADCs have the following characteristics: high therapeutic potency; high tumor cell specificity with low off-target cytotoxicity, providing a wider therapeutic window; low immunogenicity and a reduced likelihood of inducing drug resistance; extended circulation period in serum (shorter than that of naked antibodies); and weak cytotoxicity to non-target cells.

Several anti-GPC3 antibodies have been reported:

Chinese Patent CN1842540B discloses an anti-GPC3 antibody, such as GC33, which exhibits higher ADCC and CDC activities compared with conventional antibodies. Its antibody epitope is located in the sequence of 544-553 (PKDNEISTFH) at the C-terminal of GPC3, but its binding affinity for the GPC3 epitope remains relatively weak.

Chinese Patent CN10452033B discloses a high-affinity monoclonal antibody against glypican-3 and use thereof, such as YP7, which is an antibody obtained by immunization of a 50-residue peptide (DGMIKVKNQLRFLAELAYDLDVD DAPGNSQQATPKDNEISTFHNLGNVHS). It exhibits relatively high affinity for GPC3, but its affinity for GPC3 still requires further improvement.

Chinese Patent CN115850492A also discloses a monoclonal antibody, a polynucleotide, and preparation methods targeting glypican-3, and use thereof, claiming to address the technical problem of low affinity between GPC3 monoclonal antibodies and the target antigen in the prior art. Nevertheless, its affinity for GPC3 still needs further improvement.

Accordingly, the anti-GPC3 antibodies reported in the prior art still face issues such as insufficient binding affinity (especially at the cellular level), or suboptimal cytotoxicity. Therefore, there is an urgent need to develop an anti-GPC3 antibody or antigen-binding fragment with strong binding capacity to GPC3 and improved cellular cytotoxic effects.

### SUMMARY

One objective of the present invention is to provide an anti-GPC3 antibody or an antigen-binding fragment and use thereof, to address the problems of low affinity and poor cytotoxic activity in the prior art.

In one aspect, the present invention provides an anti-GPC3 antibody or an antigen-binding fragment thereof, including a heavy chain variable region (VH) and a light chain variable region (VL);

where the VH includes a heavy chain complementarity-determining region 1 (HCDR1), an HCDR2, and an HCDR3; and the HCDR1, the HCDR2, and the HCDR3 respectively include a sequence having at least 85%, 90%, 91%, 92%, 93%, 94%, 95%, 96%, 97%, 98%, 99%, or 100% identity to a CDR1, a CDR2, and a CDR3 of any one of the amino acid sequences shown in SEQ ID NOs: 1-2 and 6-9; or the HCDR1, the HCDR2, and the HCDR3 respectively include a

sequence having at most 10, 9, 8, 7, 6, 5, 4, 3, 2, or 1 mutation(s) compared with the CDR1, the CDR2, and the CDR3 of any one of the amino acid sequences shown in SEQ ID NOs: 1-2 and 6-9; the mutation(s) may be selected from insertion, deletion, and/or substitution, and the substitution is preferably a substitution of a conserved amino acid; and the CDR1, CDR2, and CDR3 regions of SEQ ID NOs: 1-2 and 6-9 are defined according to IMGT, Kabat, Chothia, AbM, or Contact; and

the VL includes a light chain complementarity-determining region 1 (LCDR1), an LCDR2, and an LCDR3; and the LCDR1, the LCDR2, and the LCDR3 respectively include a sequence having at least 85%, 90%, 91%, 92%, 93%, 94%, 95%, 96%, 97%, 98%, 99%, or 100% identity to a CDR1, a CDR2, and a CDR3 of an amino acid sequence shown in SEQ ID NO: 3; or the LCDR1, the LCDR2, and the LCDR3 respectively include a sequence having at most 10, 9, 8, 7, 6, 5, 4, 3, 2, or 1 mutation(s) compared with the CDR1, the CDR2, and the CDR3 of an amino acid sequence shown in SEQ ID NO: 3; the mutation(s) may be selected from insertion, deletion, and/or substitution, and the substitution is preferably a substitution of a conserved amino acid; and the CDR1, CDR2, and CDR3 regions of SEQ ID NO: 3 are defined according to IMGT, Kabat, Chothia, AbM, or Contact.

In some embodiments, position(s) of the mutation(s) is/are selected from one or more of a position 56 (D56), a position 100 (Q100) or a position 102 (S102) of any one of the amino acid sequences shown in SEQ ID NOs: 1-2 and 6-9. In some embodiments, position(s) of the mutation(s) include(s) a position 56 (D56) and a position 102 (S102) of any one of the amino acid sequences shown in SEQ ID NOs: 1-2 and 6-9. In some preferred embodiments, the mutation(s) is/are selected from one or more of D56A, D56K, Q100R or S102R. In some preferred embodiments, the mutation(s) is/are selected from Q100R, S102R, D56A, D56K+Q100R, D56A+S102R or D56K+S102R.

In some embodiments, the HCDR1, HCDR2, and HCDR3 respectively include a sequence identical to the CDR1, the CDR2, and the CDR3 of any one of the amino acid sequences shown in SEQ ID NOs: 1-2, 6-9, and 23-28; and the LCDR1, the LCDR2, and the LCDR3 respectively include a sequence identical to the CDR1, the CDR2, and the CDR3 of the amino acid sequence shown in SEQ ID NO: 3.

In some embodiments, the VH includes a sequence having at least 85%, 90%, 91%, 92%, 93%, 94%, 95%, 96%, 97%, 98%, 99%, or 100% identity to any one of the amino acid sequences shown in SEQ ID NOs: 1-2, 6-9, and 23-28. In some embodiments, the VL includes a sequence having at least 85%, 90%, 91%, 92%, 93%, 94%, 95%, 96%, 97%, 98%, 99%, or 100% identity to the amino acid sequence shown in SEQ ID NO: 3.

In some embodiments, the VH includes a sequence identical to any one of the amino acid sequences shown in SEQ ID NOs: 1-2, 6-9, and 23-28; and/or the VL includes a sequence identical to the amino acid sequence shown in SEQ ID NO: 3.

In some embodiments, the antibody or an antigen-binding fragment thereof further includes a heavy chain constant region (CH) and a light chain constant region (CL); where the CH may be a heavy chain constant region of human IgG1; the CL may be a light chain constant region of human x; specifically, the CH includes a sequence having at least 85%, 90%, 91%, 92%, 93%, 94%, 95%, 96%, 97%, 98%, 99%, or 100% identity to any one of the amino acid sequences shown in SEQ ID NOs: 4, 19 or 20; and the CL includes a sequence having at least 85%, 90%, 91%, 92%, 93%, 94%, 95%, 96%, 97%, 98%, 99%, or 100% identity to an amino acid sequence shown in SEQ ID NO: 5.

In some embodiments, the CH includes a sequence identical to any one of the amino acid sequences shown in SEQ ID NOs: 4, 19 or 20; and the CL includes a sequence identical to the amino acid sequence shown in SEQ ID NO: 5.

In some embodiments, the heavy chain (H) includes a sequence having at least 85%, 90%, 91%, 92%, 93%, 94%, 95%, 96%, 97%, 98%, 99%, or 100% identity to any one of the amino acid sequences shown in SEQ ID NOs: 11-18 and 29-34; and/or the light chain (L) includes a sequence having at least 85%, 90%, 91%, 92%, 93%, 94%, 95%, 96%, 97%, 98%, 99%, or 100% identity to an amino acid sequence shown in SEQ ID NO: 10.

As a preferred embodiment of the present invention, the H includes a sequence identical to any one of the amino acid sequences shown in SEQ ID NOs: 11-18 and 29-34; and the L includes a sequence identical to the amino acid sequence shown in SEQ ID NO: 10.

In some embodiments, the anti-GPC3 antibody or an antigen-binding fragment thereof of the present invention is a humanized monoclonal antibody.

In some specific embodiments, the anti-GPC3 antibody or an antigen-binding fragment thereof is: (1) a chimeric antibody or a fragment thereof; (2) a humanized antibody or a fragment thereof; or (3) a fully human antibody or a fragment thereof.

In some embodiments, the anti-GPC3 antibody or an antigen-binding fragment thereof of the present invention has one or more of the following biological functions:

(1) specific binding to an antigen shown in SEQ ID NOs: 37 or 38, and not binding to an antigen shown in SEQ ID NO: 36; and

(2) specific binding to human and Cyno GPC3 proteins, and not binding to mouse GPC3 protein.

In some embodiments, the specific binding of the anti-GPC3 antibody or an antigen-binding fragment thereof to the antigen is characterized by an equilibrium dissociation constant (KD) of, for example, $10^{-4}$M or lower (for example, $10^{-5}$M, $10^{-6}$M, $10^{-7}$M, $10^{-8}$M, $10^{-9}$M, $10^{-10}$M, $10^{-11}$M or $10^{-12}$M).

In another aspect, the present invention provides an anti-GPC3 antibody or an antigen-binding fragment thereof, where the antibody or an antigen-binding fragment thereof binds to an antigen shown in SEQ ID NOs: 37 or 38, and does not bind to an antigen shown in SEQ ID NO: 36. In some embodiments, the anti-GPC3 antibody or an antigen-binding fragment thereof includes: VL shown in SEQ ID NO: 39 and VH shown in SEQ ID NO: 40; VL shown in SEQ ID NO: 41 and VH shown in SEQ ID NO: 42; VL shown in SEQ ID NO: 43 and VH shown in SEQ ID NO: 44; VL shown in SEQ ID NO: 45 and VH shown in SEQ ID NO: 46; VL shown in SEQ ID NO: 47 and VH shown in SEQ ID NO: 48; VL shown in SEQ ID NO: 49 and VH shown in SEQ ID NO: 50; VL shown in SEQ ID NO: 51 and VH shown in SEQ ID NO: 52; VL shown in SEQ ID NO: 53 and VH shown in SEQ ID NO: 54; VL shown in SEQ ID NO: 55 and VH shown in SEQ ID NO: 56; or, VL shown in SEQ ID NO: 57 and VH shown in SEQ ID NO: 58.

In yet another aspect, the present invention provides an anti-GPC3 antibody or an antigen-binding fragment thereof, where the antibody or an antigen-binding fragment thereof competes with a reference antibody for binding to the same epitope of a GPC3 protein, where the reference antibody includes a heavy chain shown in SEQ ID NO: 11 and a light chain shown in SEQ ID NO: 10.

In some embodiments, the anti-GPC3 antibody or an antigen-binding fragment thereof inhibits the binding of the reference antibody to the GPC3 protein by at least 50%, 60%, 70%, 80%, 90%, 95%, or 99%. The competitive binding can be determined by a competitive binding assay. The competitive binding assay, well-known to those skilled in the art, is an immunological assay that detects and quantifies an unknown substance's ability to inhibit the binding of a labeled known antigen to its specific antibody. For example, an antigen is pre-coated on a microplate, and serial dilutions of an unlabeled test antibody along with a specific concentration of a labeled known antibody are added to the pre-coated microplate for incubation. After washing, an amount of known antibody bound to the plate at each dilution of the test antibody is determined. The stronger the ability of the test antibody to compete with the known antibody for antigen binding, the weaker the ability of the known antibody to bind to the antigen, and the fewer known antibodies will bind to the plate. The ability of the test antibody to block a labeled reference antibody can be determined using a radioimmunoassay, an enzyme immunoassay such as ELISA, or a fluorescence immunoassay.

Another objective of the present invention is to provide a novel GPC3 antigen epitope peptide, to address the unmet needs in the prior art for the development of anti-GPC3 antibodies, vaccines, and related diagnostic reagents, and to provide an effective tool for developing anti-GPC3 antibodies with strong binding affinity for the GPC3 or improved cytotoxic effects.

In one aspect, the present invention provides a GPC3 antigen epitope peptide, where the GPC3 antigen epitope peptide is immunogenic and capable of inducing an immune response in a body to produce antibodies against GPC3.

In another aspect, the present invention provides a GPC3 antigen epitope peptide, where the GPC3 antigen epitope peptide consists of at least 7 consecutive amino acid residues from residues 485-496 of a human GPC3 protein, and an amino acid sequence of the human GPC3 protein is shown in SEQ ID NO: 35; and the GPC3 antigen epitope peptide has one or more of the following biological functions:

(1) specifically binding to an anti-GPC3 antibody;
(2) inducing an immune response against GPC3 (e.g., a humoral immune response) in a subject;
(3) inducing the production of an anti-GPC3 antibody in a subject; and
(4) preventing and/or treating GPC3-related diseases in a subject.

In some embodiments, the specific binding of the GPC3 antigen epitope peptide to an anti-GPC3 antibody is determined using an ELISA method. In some embodiments, the ELISA method, as described in Example 12, includes coating the GPC3 antigen epitope peptide onto a microtiter plate, serially diluting the anti-GPC3 antibody, adding it to the microtiter plate for incubation, washing after incubation and developing a colorimetric reaction, terminating the reaction, and measuring an optical density at 450 nm. In some embodiments, the specific binding of the GPC3 epitope peptide to the anti-GPC3 antibody is reflected by the $OD_{450}$ value of the antigen-antibody complex as determined by ELISA. In some embodiments, when a concentration of the GPC3 antigen epitope peptide is 6 μg/mL, the $OD_{450}$ value at anti-GPC3 antibody saturation is not less than 1.5±0.1, or not less than 2 ± 0.1, or not less than 2.5 ± 0.1, or not less than 3±0.1. In some embodiments, the $OD_{450}$ value is not less than 1.5, 1.6, 1.7, 1.8, 1.9, 2.0, 2.1, 2.2, 2.2, 2.3, 2.4, 2.5, 2.6, 2.7, 2.8, 2.9, 3.0, 3.1, 3.2, 3.3, 3.4, 3.5, 3.6, 3.7, 3.8, or 3.9. In some embodiments, the anti-GPC3 antibody specifically binding to the GPC3 antigen epitope peptide includes a heavy chain and a light chain, where the heavy chain includes the amino acid sequence of SEQ ID NO: 11, and the light chain includes the amino acid sequence of SEQ ID NO: 10.

In some embodiments, the GPC3 antigen epitope peptide has a length of 7, 8, 9, 10, 11, or 12 amino acids. In some embodiments, the GPC3 antigen epitope peptide includes at least one of asparagine at a position 487 or phenylalanine at a position 493.

In some embodiments, the GPC3 antigen epitope peptide is formed by consecutive amino acid residues at positions 487-493 of the human GPC3 protein. In some preferred embodiments, the GPC3 antigen epitope peptide consists of the amino acid sequence shown in SEQ ID NO: 38.
In still another aspect, the present invention provides a recombinant antigen, including the GPC3 antigen epitope peptide of the present invention and a carrier protein. The recombinant antigen is capable of enhancing the immunogenicity of the epitope peptide, allowing it to be recognized by the immune system of a body and inducing an immune response.
In some embodiments, the recombinant antigen has one or more of the following functions:

(1) specifically binding to an anti-GPC3 antibody;
(2) inducing an immune response against GPC3 (e.g., a humoral immune response) in a subject;
(3) inducing the production of an anti-GPC3 antibody in a subject; and
(4) preventing and/or treating GPC3-related diseases in a subject.

In some embodiments, the anti-GPC3 antibody specifically binding to the recombinant antigen includes a heavy chain and a light chain, where the heavy chain includes the amino acid sequence of SEQ ID NO: 11, and the light chain includes the amino acid sequence of SEQ ID NO: 10.
In some embodiments, the GPC3 antigen epitope peptide of the present invention is directly linked to or linked via a linker to the carrier protein. In some embodiments, the linker may be a rigid or flexible linker, such as a peptide linker, where the peptide linker includes one or more serine and/or glycine residues.
In some embodiments, the GPC3 antigen epitope peptide of the present invention is linked to an N-terminus and/or C-terminus, and/or inserted into an interior of the carrier protein. In some preferred embodiments, the GPC3 antigen epitope peptide is linked to the C-terminus of the carrier protein. In some preferred embodiments, the GPC3 antigen epitope peptide is linked to the N-terminus of the carrier protein.
In some embodiments, the carrier protein includes, but is not limited to, keyhole limpet hemocyanin (KLH), bovine serum albumin (BSA), thyroglobulin, fibrinogen, gelatin, multiple antigen peptide, diphtheria toxin (DT), a transmembrane domain of diphtheria toxin (DTT), rotavirus viral protein 7 (VP7), heat shock protein of *Leishmania,* flagellin of *Campylobacter jejuni,* major outer membrane protein of *Chlamydia trachomatis,* chicken ovalbumin (OVA), or an immunoglobulin Fc domain, such as the Fc domain of IgG1, IgG2, IgG3, or IgG4. In some preferred embodiments, the carrier protein is selected from KLH and BSA.
In still another aspect, the present invention provides a chimeric antigen receptor, which includes the anti-GPC3 antibody or an antigen-binding fragment thereof.
In still another aspect, the present invention provides a chimeric antigen receptor (CAR), which includes an extracellular antigen-binding domain, a transmembrane domain, and an intracellular signaling domain, where the extracellular antigen-binding domain includes the anti-GPC3 antibody or an antigen-binding fragment thereof.
In still another aspect, the present invention further provides a pharmaceutical composition including the anti-GPC3 antibody or an antigen-binding fragment thereof of the present invention, or an antibody conjugate (such as an antibody-drug conjugate), or an oncolytic virus, or a chimeric antigen receptor, or a bispecific or multispecific antibody molecule, or a GPC3 antigen epitope peptide, or a recombinant antigen, or one or more pharmaceutically acceptable carriers. When the composition includes more than one antibody (or antigen-binding fragments thereof, or antibody conjugates, or oncolytic viruses), the antibodies (or the antigen-binding fragments thereof, or the antibody conjugates, or the oncolytic viruses) may be administered in batches. The composition may optionally include one or more additional pharmacologically active agents, such as another antibody or drug, for example, an anti-tumor drug.
The pharmaceutical composition of the present invention may be a vaccine, including but not limited to a protein vaccine or a nucleic acid vaccine.
The pharmaceutical composition may include any number of excipients. Usable excipients include carriers, surfactants, thickeners or emulsifiers, solid binders, dispersing or suspending agents, solubilizers, coloring agents, flavoring agents, coating agents, disintegrants, lubricants, sweeteners, preservatives, isotonic agents, or combinations thereof. Guidance for selecting and using suitable excipients is provided in the following literature, edited by Gennaro, Remington: The Science and Practice of Pharmacy, 20th edition (Lippincott Williams & Wilkins, 2003), the disclosure of which is incorporated herein by reference.
In still another aspect, the present invention provides a kit, including the anti-GPC3 antibody or an antigen-binding fragment thereof, the GPC3 antigen epitope peptide, the recombinant antigen, the antibody-drug conjugate, or the bispecific or multispecific antibody molecule of the present invention.
In some embodiments, the kit includes the GPC3 antigen epitope peptide of the present invention and tools for detecting antibodies.
In some embodiments, the kit is used to detect the anti-GPC3 antibodies. In some embodiments, the kit is used to detect the presence of anti-GPC3 antibodies in a sample. In some embodiments, the kit is used to detect a level of anti-GPC3 antibodies in a sample. In some embodiments, the anti-GPC3 antibody includes a heavy chain and a light chain, where the heavy chain includes the amino acid sequence of SEQ ID NO: 11, and the light chain includes the amino acid sequence of SEQ ID NO: 10.

In still another aspect, the present invention provides a polynucleotide encoding the anti-GPC3 antibody or an antigen-binding fragment thereof, the GPC3 antigen epitope peptide, or the recombinant antigen of the present invention. The polynucleotide of the present invention may be, for example, DNA or RNA, and may or may not contain intron sequences. In a preferred embodiment, the polynucleotide is a cDNAmolecule. The polynucleotide of the present invention may be prepared or obtained based on the amino acid sequence information of the present invention by known methods, such as by automated DNA synthesis and/or recombinant DNA technology.

As is well known in the art, multiple codons can encode the same amino acid. Accordingly, nucleic acids encoding a protein sequence may include nucleic acids having codon degeneracy. The amino acid sequences of the present invention may be encoded by a variety of nucleic acids. The genetic code is universal and well known. Nucleic acids encoding any amino acid sequence of the present invention can be readily designed based on common general knowledge by those skilled in the art and can be optimized for production. While the possible number of nucleic acid sequence encoding a given amino acid sequence is large, given a standard table of the genetic code, and aided by a computer, those skilled in the art can readily generate every possible combination of nucleic acids encoding a given amino acid sequence.

In still another aspect, the present invention provides an expression vector, which includes the polynucleotide of the present invention. The expression vector includes bacterial plasmids, bacteriophages, yeast plasmids, plant cell viruses, mammalian cell viruses such as adenoviruses, retroviruses, or other vectors.

In still another aspect, the present invention provides a host cell, which includes the polynucleotide or the expression vector of the present invention, where the host cell includes a prokaryotic cell, a yeast cell, or a mammalian cell, such as CHO cells, NS0 cells, or other mammalian cells, preferably CHO cells.

In still another aspect, the present invention provides a bispecific or multispecific antibody molecule, which includes the anti-GPC3 antibody or an antigen-binding fragment thereof.

In still another aspect, the present invention provides an antibody-drug conjugate (ADC), which includes the anti-GPC3 antibody or an antigen-binding fragment thereof of the present invention and a drug or a toxin. The drug or toxin is selected from one or more of SN-38, MMAE, PBD dimer, DX-8951 (DXd), or DUBA.

The antibody and the drug may be conjugated via a linker to form the antibody-drug conjugate. Typically, the ADC includes the anti-GPC3 antibody or an antigen-binding fragment thereof of the present invention, and linked to the drug or the toxin via a linker. The linker may be cleavable or non-cleavable. Cleavable linkers are typically susceptible to cleavage in an intracellular environment, thereby releasing the therapeutic agent from the antibody. Suitable cleavable linkers include enzymatically cleavable linkers, such as peptide-containing linkers capable of being cleaved by intracellular lysosomal proteases, or glycosidic linkers, such as glucuronide-containing linkers capable of being cleaved by glucuronidase. Peptide linkers may include dipeptides, such as valine-citrulline, phenylalanine-lysine, or valine-alanine. Other suitable cleavable linkers include pH-sensitive linkers (e.g., hydrazone linkers hydrolyzed at pH below 5.5) and linkers cleavable under reducing conditions (e.g., disulfide linkers). Non-cleavable linkers typically release the drug under conditions where the antibody is hydrolyzed by proteases.

Prior to conjugation to the antibody, the linker has an active reactive group capable of reacting with some amino acid residues, and the conjugation is achieved via the active reactive group. Thiol-specific reactive groups are preferred, such as maleimide compounds, haloamides, haloesters, halomethyl ketones, benzyl halides, vinyl sulfones, pyridyl disulfides, mercury derivatives, and polymethylene dimethyldithiophosphate sulfonates. The linker may include, for example, a maleimide linked to the antibody via thiosuccinimide.

Preferably, the drug or the toxin linked via the linker is selected from CL2A-SN-38 (CAS No.: 1279680-68-0), mc-vc-PAB-MMAE (CAS No.: 646502-53-6), Tesirine (SG3249, CAS No.: 1595275-62-9), Deruxtecan (CAS No.: 1599440-13-7), or Vc-seco-DUBA (SYD985, CAS No.: 1345681-58-4). Molecular structures are illustrated in the figures below:

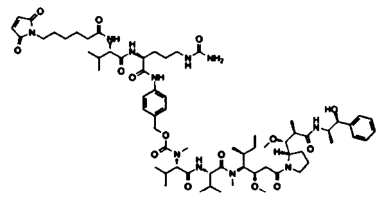

mc-vc-PAB-MMAE

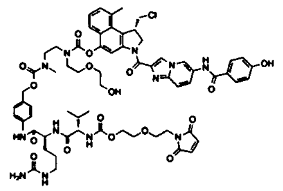

Vc-seco-DUBA

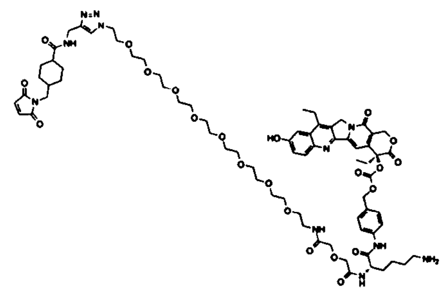

CL2A-SN-38

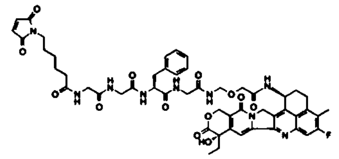

Deruxtecan

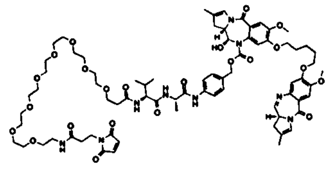

Tesirine

In the present invention, the anti-GPC3 antibody or an antigen-binding fragment thereof is conjugated to SN-38 via a CL2A linker.

In the present invention, the anti-GPC3 antibody or an antigen-binding fragment thereof is conjugated to MMAE via an mc-VC-PAB linker.

In the present invention, the anti-GPC3 antibody or an antigen-binding fragment thereof is conjugated to a PBD dimer via a maleimide-dPEG8-VA-PABA linker.

Preferably, the drug may be any cytotoxic, cell growth inhibitory, or immunosuppressive agent. In some embodiments, the linker connects the antibody and the drug, and the drug includes a functional group capable of forming a bond with the linker. For example, the drug may have an amino, carboxyl, thiol, hydroxyl, or keto group capable of bonding with the linker. When the drug is directly linked to the linker, the drug has a reactive group prior to being linked to the antibody.

Preferably, the cytotoxic drug is selected from the group consisting of anti-microtubule agents, DNA minor groove binders, DNA replication inhibitors, DNA alkylating agents, antibiotics, folic acid antagonists, antimetabolites, chemosensitizers, topoisomerase inhibitors, vinca alkaloids, or combinations thereof.

Preferably, particularly useful examples of cytotoxic drugs include, for example, DNA minor groove binders, DNA alkylating agents and anti-microtubule agents. Typical cytotoxic drugs include, for example, auristatins, camptothecins, duocarmycins, etoposides, maytansines and maytansinoids (e.g., DM1 and DM4), taxanes, benzodiazepines or benzodiazepine containing drugs (e.g., pyrrolo[1,4]benzodiazepines (PBDs)), indolinobenzodiazepines, and oxazolidinobenzodiazepines, vinca alkaloids, or combinations thereof.

Preferably, the toxin is selected from the group consisting of auristatins (e.g., auristatin E, auristatin F, MMAE, and MMAF), chlortetracycline, exemestane, ricin, ricin A-chain, combretastatin, amikacin, dolastatin, doxorubicin, daunorubicin, paclitaxel, cisplatin, cc1065, ethidium bromide, mitomycin, etoposide, tenoposide, vincristine, vinblastine, colchicine, dihydroxyanthraxetine dione, actinomycin, diphtheria toxin, *Pseudomonas exotoxin* (PE) A, PE40, abrin, abrin A-chain, modeccin A-chain, $\alpha$-hemolytic streptococci, cerberin, mitogellin, restrictocin, phenomycin, ivermectin, curicin, croton toxin, calicheamicin, *Saponaria officinalis* inhibitors, glucocorticoids, or combinations thereof.

Preferably, the drug or the toxin is selected from one or more of SN-38 (NK012, CAS No.: 86639-52-3), MMAE (Monomethyl auristatin E, CAS No.: 474645-27-7), PBD dimer (SG3199, CAS No.: 1595275-71-0), DX-8951 (Exatecan, CAS No.: 171335-80-1), or DUBA (duocarmycin-hydroxybenzamide-azaindole).

In the present invention, the anti-GPC3 antibody or an antigen-binding fragment thereof is conjugated to DX-8951 (DXd) via a maleimide-GGFG linker.

In the present invention, the anti-GPC3 antibody or an antigen-binding fragment thereof is conjugated to DUBA via a Vc-seco linker.

In still another aspect, the present invention provides use of the anti-GPC3 antibody or an antigen-binding fragment thereof, the pharmaceutical composition of the present invention, or the antibody-drug conjugate of the present invention in the preparation of a medicament for treating or preventing cancer, where the cancer is preferably liver cancer.

In another aspect, the present invention provides use of the GPC3 antigen epitope peptide, the recombinant antigen, the nucleic acid molecule, vector, or the host cell of the present invention in any one of the following:

(1) preparing an anti-GPC3 antibody or an antigen-binding fragment thereof;
(2) preparing a product for treating and/or preventing and/or diagnosing GPC3-related diseases in a subject, and preferably, the diseases are a GPC3-positive cancer;

(3) preparing a product for detecting an anti-GPC3 antibody or an antigen-binding fragment thereof; and preferably the anti-GPC3 antibody includes the heavy chain shown in SEQ ID NO: 11 and the light chain shown in SEQ ID NO: 10;
(4) detecting an anti-GPC3 antibody or an antigen-binding fragment thereof; and preferably the anti-GPC3 antibody includes the heavy chain shown in SEQ ID NO: 11 and the light chain shown in SEQ ID NO: 10; and
(5) screening for an anti-GPC3 antibody or an antigen-binding fragment thereof.

In some embodiments, the disease is a GPC3-positive cancer, such as liver cancer, colorectal cancer, ovarian cancer, and the like.

In still another aspect, the present invention provides a method for preparing an anti-GPC3 antibody or an antigen-binding fragment thereof, including the step of stimulating an animal's immune system with a GPC3 antigen epitope peptide, the recombinant antigen, the nucleic acid molecule, the vector, or the host cell of the present invention, thereby causing the animal to produce antibodies.

In some embodiments, the animal is selected from mammals such as humans, mice, rabbits, monkeys, cows, sheep, or alpacas.

In still another aspect, the present invention provides a method for screening for an anti-GPC3 antibody or an antigen-binding fragment thereof, including contacting the GPC3 antigen epitope peptide of the present invention with the antibody or an antigen-binding fragment thereof to be analyzed, and detecting binding of the GPC3 antigen epitope peptide to the antibody or antigen-binding fragment thereof. When binding is observed between the GPC3 antigen epitope peptide and the antibody or an antigen-binding fragment thereof, the antibody or an antigen-binding fragment thereof is identified as a candidate anti-GPC3 antibody or an antigen-binding fragment thereof.

In some embodiments, the anti-GPC3 antibody or an antigen-binding fragment thereof binds to the GPC3 antigen epitope peptide, and the binding is measured by an ELISA method. In some embodiments, the ELISA method, as described in Example 12, includes coating the GPC3 antigen epitope peptide onto a microtiter plate, serially diluting the anti-GPC3 antibody or an antigen-binding fragment thereof, adding it to the microtiter plate for incubation, washing after incubation and developing a colorimetric reaction, terminating the reaction, and measuring an optical density at 450 nm. In some embodiments, the specific binding of the anti-GPC3 antibody or an antigen-binding fragment thereof to the anti-GPC3 antibody is reflected by the $OD_{450}$ value of the antigen-antibody complex as determined by ELISA. In some embodiments, when a concentration of the GPC3 antigen epitope peptide is 6 $\mu$g/mL, the $OD_{450}$ value at saturation of the anti-GPC3 antibody or an antigen-binding fragment thereof is not less than 1.5$\pm$0.1, or not less than 2 $\pm$ 0.1, or not less than 2.5 $\pm$ 0.1, or not less than 3 $\pm$ 0.1. In some embodiments, the $OD_{450}$ value is not less than 1.5, 1.6, 1.7, 1.8, 1.9, 2.0, 2.1, 2.2, 2.2, 2.3, 2.4, 2.5, 2.6, 2.7, 2.8, 2.9, 3.0, 3.1, 3.2, 3.3, 3.4, 3.5, 3.6, 3.7, 3.8, or 3.9. Further, the present invention provides a method for preparing the anti-GPC3 antibody or an antigen-binding fragment of the present invention.

The DNA sequence of the anti-GPC3 antibody or an antigen-binding fragment of the present invention may be obtained using conventional techniques, such as hybridoma PCR amplification or phage display library screening. In addition, the coding sequences of the heavy and light chains may be fused to form a single-chain antibody (e.g., scFv).

Once being obtained, the relevant sequence may be cloned into a vector, introduced into a host cell, and the vector can be extracted from the host cell using conventional methods.

Moreover, the relevant sequence may be synthesized artificially, especially when the fragment length is short. Currently, DNA sequence encoding the antibody (or a fragment thereof or a derivative thereof) of the present invention can be entirely obtained through chemical synthesis. Further, mutations can be introduced into the protein sequence of the present invention through chemical synthesis.

The present invention further relates to vectors including the abovementioned appropriate DNA sequence and suitable promoters or control sequences. These vectors can be used to transform suitable host cells to enable protein expression.

The anti-GPC3 antibody or an antigen-binding fragment thereof of the present invention may be expressed intracellularly, on the cell membrane, or secreted extracellularly. If desired, the recombinant protein may be isolated and purified using various isolation methods based on its physical, chemical, and other properties. These methods are well known to those skilled in the art. Typically, the transformed host cells are cultured under conditions suitable for antibody expression, and then purified to obtain the anti-GPC3 antibody and an antigen-binding fragment thereof of the present invention using conventional immunoglobulin purification steps, such as Protein A-Sepharose affinity chromatography, ion exchange chromatography, hydrophobic chromatography, molecular sieve chromatography, hydroxyapatite chromatography, gel electrophoresis, dialysis, or combinations thereof.

In a preferred embodiment of the method for preparing the anti-GPC3 antibody or an antigen-binding fragment thereof, the method for separating and purifying the anti-GPC3 antibody or an antigen-binding fragment thereof is performed using the Protein A affinity chromatography, cation exchange chromatography, or anion exchange chromatography.

The resulting monoclonal antibody or bispecific antibody may be identified using conventional methods. For example, the binding specificity of the antibody may be determined by immunoprecipitation or in vitro binding assays, such

as enzyme-linked immunosorbent assay (ELISA) or radioimmunoassay (RIA). The binding affinity of the antibody may be determined, for example, using Scatchard assay as described by Munson et al., Anal. Biochem., 107: 220 (1980).

In the present invention, the antibody-drug conjugate is prepared by a method including the following steps:

reducing the interchain disulfide bonds of the anti-GPC3 antibody or an antigen-binding fragment thereof of the present invention to generate 2n (e.g., 2, 4, 6, 8) reactive thiol groups;
crosslinking the drug-linker compound to the reduced antibody to generate the corresponding antibody-drug conjugate; and
performing further ultrafiltration and desalting to obtain the product.

For purposes of clarity, the present invention defines common terms used in the description of the compounds herein.

Unless otherwise indicated, the following terms and phrases as used in the present invention are intended to have the meanings set forth below. A particular term or phrase, unless specifically defined, should not be considered indefinite or unclear in the absence of an explicit definition, but rather should be interpreted according to its ordinary and customary meaning in the art. When trade names appear herein, they are intended to refer to the corresponding commercial products or their active ingredients. As used herein, the term "pharmaceutically acceptable" refers to the compounds, materials, compositions, and/or dosage forms that, within the scope of sound medical judgment, are suitable for contact with human or animal tissues without excessive toxicity, irritation, allergic reaction, or other problems or complications, and that have an appropriate benefit-to-risk ratio.

GPC3 (also known as glypican-3) is a member of the heparan sulfate proteoglycan family, anchored to the cell surface via a glycosyl-phosphatidylinositol (GPI) moiety. The human GPC3 gene is located on the X chromosome (Xp26) and encodes a 70 kDa protein with 580 amino acids. This protein is cleaved by a furin-like convertase between Arg358 and Ser359, resulting in a 40 kDa N-terminal subunit and a 30 kDa C-terminal subunit, with two heparan sulfate (HS) chains on the C-terminal subunit.

As used herein, the term "antibody" includes a whole antibody and any antigen-binding fragment (i.e., "antigen binding portion") or single chains thereof. The whole antibody is a glycoprotein consisting of two heavy (H) chains and two light (L) chains linked by disulfide bonds. Each heavy chain contains a heavy chain variable region (abbreviated VH herein) and a heavy chain constant region. The heavy chain constant region consists of three domains CH1, CH2, and CH3. Each light chain contains a light chain variable region (abbreviated VL herein) and a light chain constant region. The light chain constant region is composed of one domain, CL. The VH and VL regions can be further subdivided into regions of hypervariability, termed complementarity determining regions (CDRs), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "antigen-binding fragment" (or simply "antibody portion") refers to one or more fragments of an antibody that retain the ability to specifically bind to an antigen (e.g., a GPC3 protein). It has been demonstrated that the antigen-binding function of an antibody can be performed by fragments of a full-length antibody. Examples of binding fragments encompassed within the term "antigen-binding fragment" of an antibody include (i) a Fab fragment, a monovalent fragment consisting of the VL VH, CL and CH1 domains; (ii) a F(ab')2 fragment, a bivalent fragment including two Fab fragments linked by a disulfide bridge at the hinge region; (iii) a Fd fragment consisting of the VH and CH1 domains; (iv) a Fv fragment consisting of the VL and VH domains of a single arm; (v) a dAb fragment (Ward et al., (1989) Nature 341: 544-546), which consists of a VH domain; (vi) an isolated complementarity determining region (CDR); and (vii) a nanobody, a heavy chain variable region containing a single variable domain and two constant domains. Furthermore, although the two domains of the Fv fragment, VL and VH, are coded by separate genes, they can be linked by a linker using a recombinant method, enabling them to be made as a single protein chain in which the VL and VH regions pair to form a monovalent molecule (known as a single chain Fv (scFv); see e.g., Bird et al. (1988), Science 242: 423-426; and Huston et al. (1988), Proc. Natl. Acad. Sci., USA 85: 5879-5883). Such single chain antibodies are also intended to be encompassed within the scope of "antigen-binding fragment" of the antibody. These antibody fragments are obtained using conventional techniques known to those skilled in the art, and the fragments are screened for use in the same manner as are intact antibodies.

As used herein, the term "isolated antibody" refers to an antibody that is substantially free of other antibodies having different antigenic specificities. For example, an isolated antibody that specifically binds a GPC3 protein is substantially free of antibodies that specifically bind antigens other than GPC3. An isolated antibody that specifically binds a human GPC3 protein may, however, have cross-reactivity to other antigens, such as GPC3 proteins from other species. Moreover, an isolated antibody can be substantially free of other cellular material and/or chemicals.

As used herein, the term "monoclonal antibody" refers to a preparation of antibody molecules of single molecular

composition. A monoclonal antibody composition displays a single binding specificity and affinity for a particular epitope.

The term "chimeric antibody" refers to an antibody made by combining genetic material from a nonhuman source with genetic material from a human being. Or more generally, a chimeric antibody is an antibody having genetic material from a specific species with genetic material from another species.

As used herein, the term "bispecific" or "multispecific" refers to an antibody and/or an antigen-binding molecule capable of specifically binding two or more different antigenic determinants. Typically, a bispecific or multispecific antibody or antigen-binding molecule includes two antigen binding sites, each of which is specific for a different antigenic determinant. In some embodiments, the bispecific or multispecific antibody or antigen-binding molecule is capable of simultaneously binding two or more antigenic determinants, particularly antigenic determinants expressed on two or more distinct cell types.

As used herein, the term "humanized antibody" refers to an antibody originating from a non-human species whose protein sequence has been modified to increase its similarity to naturally occurring human antibody variants.

As used herein, the term "antibody-drug conjugate (ADC)" refers to a drug that utilizes the specific recognition of tumor cell surface antigens by antibodies to precisely deliver antitumor therapeutic agents (such as cytotoxins, cytostatic agents, radionuclides, or small-molecule chemotherapeutics) to tumor target cells, causing intracellular accumulation and release, thereby achieving precise tumor killing. Due to their optimal molecular size, high stability, strong target specificity, and reduced toxicity, ADCs are considered among the most promising classes of antitumor drugs. In addition to monoclonal antibodies, bispecific antibodies can also be coupled to therapeutic agents. In some embodiments, the portion conjugated to the antibody or bispecific antibody of the present invention to form an antibody conjugate is a cytotoxin, which is a substance that inhibits or prevents cellular function and/or induces cell damage, including small-molecule cytotoxins. In some embodiments, the cytotoxin is selected from SN-38, MMAE, PBD dimer, DX-8951 (DXd) or DUBA.

The terms "comprising/comprise," "including/include," and "having/have" are intended to be inclusive and mean that there may be additional elements other than those explicitly listed. It should also be understood that descriptions employing "comprising/comprise," "including/include," and "having/have" likewise provide support for embodiments described using "consisting of."

As used herein, the term "antibody" includes, but is not limited to, monoclonal antibodies, polyclonal antibodies, monospecific antibodies, multispecific antibodies (such as bispecific antibodies), monovalent antibodies, multivalent antibodies, full-length antibodies, antigen-binding fragments, unconjugated ("naked") antibodies, conjugated antibodies, humanized antibodies, and fully human antibodies.

As used herein, the term "epitope" refers to the portion of an antigen specifically bound by an immunoglobulin or antibody. The term "epitope" is also referred to in the art as an "antigenic determinant." An epitope or an antigenic determinant typically consists of chemically active surface groups of a molecule such as amino acids, carbohydrates, or glycan side chains, and generally has specific three-dimensional structural and electrostatic features. For example, an epitope may include at least 3, 4, 5, 6, 7, 8, 9, 10, 11, 12, 13, 14, or 15 contiguous or non-contiguous amino acids arranged in a unique spatial conformation, and may be "linear" or "conformational." See, e.g., Epitope Mapping Protocols in Methods in Molecular Biology, Vol. 66, G. E. Morris, Ed. (1996). In a linear epitope, all contact points between the protein and the interacting molecule (such as an antibody) occur linearly along the primary amino acid sequence of the protein. In a conformational epitope, all contact points occur across amino acid residues on the protein that are separated from one another.

As used herein, the term "epitope peptide" refers to a peptide on an antigen that can function as an epitope. In some cases, an isolated epitope peptide may be specifically recognized and/or bound by an antibody directed to the corresponding epitope. In other cases, the epitope peptide may need to be fused to a carrier protein, so that the epitope peptide can be recognized by a specific antibody.

As used herein, the term "carrier protein" refers to such a protein that may act as a carrier of epitope peptide, i.e. the epitope peptide may be inserted into the protein at a specific position (for example, inside the protein, N-terminal or C-terminal of the protein), so that the epitope peptide can be presented and thus can be recognized by an antibody or immune system.

As used herein, the term "conservative amino acid" generally refers to amino acids belonging to the same class of residues or having similar characteristics (such as, charge, side-chain size, hydrophobicity, hydrophilicity, backbone conformation, or rigidity). For example, the amino acids in each of the following groups as conserved amino acid residues, and substitutions of amino acid residues within a group are considered conserved amino acid substitutions:

1) alanine (A), serine (S), threonine (T);
2) aspartic acid (D), glutamic acid (E);
3) asparagine (N), glutamine (Q);
4) arginine (R), lysine (K), histidine (H);
5) isoleucine (I), leucine (L), methionine (M), valine (V); and

6) phenylalanine (F), tyrosine (Y), tryptophan (W).

As used herein, the terms "identity" and "sequence similarity" are interchangeable and are determined as follows: to determine the identity percentage of two amino acid sequences or two nucleic acid sequences, the sequences are aligned for optimal comparison (e.g., gaps can be introduced in the first amino acid or nucleic acid sequence to be compared with the second amino acid sequence or nucleic acid sequence for optimal alignment, or non-homologous sequences may be discarded for comparison purposes). The corresponding amino acid residues or nucleotides in the aligned sequences are then compared. When a position in the first sequence is occupied by the same amino acid residue or nucleotide at the corresponding position in the second sequence, these molecules are identical at this position.

As used herein, the symbol "+" indicates a combination of mutations. As used herein, the following terminology is used to denote mutations: "S102R" indicates that at a position 102 of the parental sequence, the amino acid residue serine (S) is substituted with arginine (R).

The term "subject" refers to any human or non-human animal. The term "non-human animal" includes all vertebrates, such as mammalian and non-mammalian species, for example, non-human primates, rodents, rabbits, pigs, dogs, cats, chickens, amphibians, and reptiles, although mammals, particularly non-human primates and rodents, are preferred.

The term "therapeutically effective amount" refers to an amount of the anti-GPC3 antibody or an antigen-binding fragment thereof of the present invention sufficient to prevent or ameliorate one or more symptoms associated with a disease or disorder (e.g., cancer) and/or to reduce the severity of the disease or disorder. The therapeutically effective amount should be understood in the context of the disease being treated, and those skilled in the art will readily recognize an actual effective amount. The antibodies of the present invention are monoclonal antibodies that are structurally and chemically characterized as described herein and in the following examples. The amino acid sequences of the heavy/light chain variable regions and constant regions of the antibodies (SEQ ID NOs: ) are summarized in Tables 1 and 5.

The heavy chain variable-region CDRs and light chain variable-region CDRs in Table 1 and Table 5 are defined according to the Kabat, Chothia, IMGT, AbM, or Contact numbering systems/methods. Exemplary CDR sequences of the anti-GPC3 antibodies of the present invention are itemized in Table 3.

Table 1. Amino acid sequences of the heavy/light chain variable regions and constant regions of anti-GPC3 antibodies

| Antibody | SEQ ID NO: | Amino acid sequence |
|---|---|---|
| GC90-VH | 1 | QVQLLESGGGLVQPGGSLRVSCAASGFTFNSYAMSWVRQAPGKGLE WVASIKSSGDRVQYADSVKGRFTISRDNSKNTVYLEMNSLRAEDTAIY YCAKPQWSSPQRTFEVWGQGTPVTVSS |
| GC90LH5-VH | 2 | EVQLLESGGGLVQPGGSLRLSCAASGFTFNSYAMSWVRQAPGKGLE WVASIKSSGDRVQYADSVKGRFTISRDNSKNTVYLQMNSLRAEDTAI YYCAKPQWSSPQRTFEVWGQGTMVTVSS |
| GC90-VL | 3 | DIQLTQSPSSLSASVGDRVTITCRASQSISSYLNWYQQKPGKAPKLLIY AASSLQSGVPSRFSGSGSGTDFTLTISSLQPEDFATYYCQQSYSTPWTF GQGTKVEIK |
| GC90-CH | 4 | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK RVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTV LHQDWLNGKEYKCKVSNKALPAPIEKTISKAKGQPREPQVYTLPPSR DELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| GC90-CL | 5 | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| GC90LH1-VH | 6 | EVQLLESGGGLVQPGGSLRLSCAASGFTFNSYAMSWVRQAPGKGLE WVASIKSSGDRVQYADSVKGRFTISRDNSKNTVYLEMNSLRAEDTAIY YCAKPQWSSPQRTFEVWGQGTPVTVSS |

(continued)

| Antibody | SEQ ID NO: | Amino acid sequence |
|---|---|---|
| GC90LH2-VH | 7 | QVQLLESGGGLVQPGGSLRVSCAASGFTFNSYAMSWVRQAPGKGLEWVASIKSSGDRVQYADSVKGRFTISRDNSKNTVYLQMNSLRAEDTAIYYCAKPQWSSPQRTFEVWGQGTPVTVSS |
| GC90LH3-VH | 8 | QVQLLESGGGLVQPGGSLRVSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTMVTVSS |
| GC90LH4-VH | 9 | EVQLLESGGGLVQPGGSLRLSCAASGFTFNSYAMSWVRQAPGKGLEWVASIKSSGDRVQYADSVKGRFTISRDNSKNTVYLQMNSLRAEDTAIYYCAKPQWSSPQRTFEVWGQGTPVTVSS |
| GC90-L | 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| GC90-H | 11 | QVQLLESGGGLVQPGGSLRVSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| GC90-4mu-H | 12 | QVQLLESGGGLVQPGGSLRVSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGP**C**VFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| GC90-6mu-H | 13 | QVQLLESGGGLVQPGGSLRVSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGP**C**VFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |

(continued)

| Antibody | SEQ ID NO: | Amino acid sequence |
|---|---|---|
| GC90LH1-H | 14 | EVQLLESGGGLVQPGGSLRLSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| GC90LH2-H | 15 | QVQLLESGGGLVQPGGSLRVSCAASGFTFNSYAMSWVRQAPGKGLEWVASIKSSGDRVQYADSVKGRFTISRDNSKNTVYLQMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| GC90LH3-H | 16 | QVQLLESGGGLVQPGGSLRVSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| GC90LH4-H | 17 | EVQLLESGGGLVQPGGSLRLSCAASGFTFNSYAMSWVRQAPGKGLEWVASIKSSGDRVQYADSVKGRFTISRDNSKNTVYLQMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| GC90LH5-H | 18 | EVQLLESGGGLVQPGGSLRLSCAASGFTFNSYAMSWVRQAPGKGLEWVASIKSSGDRVQYADSVKGRFTISRDNSKNTVYLQMNSLRAEDTAIYYCAKPQWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| Antibody | SEQ ID NO: | Amino acid sequence |
|---|---|---|
| GC90-4muCH | 19 | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK RVEPKSCDKTHTCPPCPAPEAAGGPCVFLFPPKPKDTLMISRTPEVTCV VVDVSHEDPEVKFNWYVDGVEVHNAKTKPREEQYNSTYRVVSVLTV LHQDWLNGKEYKCKVSNKALGAPIEKTISKAKGQPREPQVYTLPPSR DELTKNQVSLTCLVKGFYPSDIAVEWESNGQPENNYKTTPPVLDSDGS FFLYSKLTVDKSRWQQGNVFSCSVMHEALHNHYTQKSLSLSPGK |
| GC90-6muCH | 20 | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK RVEPKSCDKTHTCPPCPAPEAAGGPCVFLFPPKPKDTLMISRTPEVTCV VVDVSHEDPEVKFNWYVDGVEVHNAKTKPREEQYNSTYRVVSVLTV LHQDWLNGKEYKCKVSNKALGAPIEKTISKAKGQPREPQVYTLPPSR DELTKNQVSLTCLVKGFYPSDIAVEWESNGQPENNYKTTPPVLDSDGS FFLYSKLTVDKSRWQQGNVFSCSVLHEALHSHYTQKSLSLSPGK |
| hYP7HM-L | 21 | DIVMTQSPDSLAVSLGERATINCKSSQSLLYSSNQKNYLAWYQQKPG QPPKLLIYWASSRESGVPDRFSGSGSGTDFTLTISSLQAEDVAVYYCQQ YYNYPLTFGQGTKLEIKRTVAAPSVFIFPPSDEQLKSGTASVVCLLNNF YPREAKVQWKVDNALQSGNSQESVTEQDSKDSTYSLSSTLTLSKADY EKHKVYACEVTHQGLSSPVTKSFNRGEC |
| hYP7HM-H | 22 | EVQLVESGGGLVQPGGSLRLSCAASGFTFNKNAMNWVRQAPGKGLE WVGRIRNKTNNYATYYADSVKGRFTISRDDSKNSLYLQMNSLKTEDT AVYYCVAGNSFAYWGQGTLVTVSASTKGPSVFPLAPSSKSTSGGTAA LGCLVKDYFPEPVTVSWNSGALTSGVHTFPAVLQSSGLYSLSSVVTVP SSSLGTQTYICNVNHKPSNTKVDKRVEPKSCDKTHTCPPCPAPEAAGG PCVFLFPPKPKDTLMISRTPEVTCVVVDVSHEDPEVKFNWYVDGVEV HNAKTKPREEQYNSTYRVVSVLTVLHQDWLNGKEYKCKVSNKALG APIEKTISKAKGQPREPQVYTLPPSRDELTKNQVSLTCLVKGFYPSDIA VEWESNGQPENNYKTTPPVLDSDGSFFLYSKLTVDKSRWQQGNVFSC SVLHEALHSHYTQKSLSLSPGK |
| GC90LH6-VH | 23 | EVQLLESGGGLVQPGGSLRLSCAASGFTFNSYAMSWVRQAPGKGLE WVASIKSSGDRVQYADSVKGRFTISRDNSKNTVYLQMNSLRAEDTAI YYCAKPRWSSPQRTFEVWGQGTMVTVSS |
| GC90LH7-VH | 24 | EVQLLESGGGLVQPGGSLRLSCAASGFTFNSYAMSWVRQAPGKGLE WVASIKSSGDRVQYADSVKGRFTISRDNSKNTVYLQMNSLRAEDTAI YYCAKPQWRSPQRTFEVWGQGTMVTVSS |
| GC90LH8-VH | 25 | EVQLLESGGGLVQPGGSLRLSCAASGFTFNSYAMSWVRQAPGKGLE WVASIKSSGARVQYADSVKGRFTISRDNSKNTVYLQMNSLRAEDTAI YYCAKPQWSSPQRTFEVWGQGTMVTVSS |
| GC90LH9-VH | 26 | EVQLLESGGGLVQPGGSLRLSCAASGFTFNSYAMSWVRQAPGKGLE WVASIKSSGKRVQYADSVKGRFTISRDNSKNTVYLQMNSLRAEDTAI YYCAKPRWSSPQRTFEVWGQGTMVTVSS |
| GC90LH10-VH | 27 | EVQLLESGGGLVQPGGSLRLSCAASGFTFNSYAMSWVRQAPGKGLE WVASIKSSGARVQYADSVKGRFTISRDNSKNTVYLQMNSLRAEDTAI YYCAKPQWRSPQRTFEVWGQGTMVTVSS |
| GC90LH 11-VH | 28 | EVQLLESGGGLVQPGGSLRLSCAASGFTFNSYAMSWVRQAPGKGLE WVASIKSSGKRVQYADSVKGRFTISRDNSKNTVYLQMNSLRAEDTAI YYCAKPQWRSPQRTFEVWGQGTMVTVSS |

(continued)

| Antibody | SEQ ID NO: | Amino acid sequence |
|---|---|---|
| GC90LH6-H | 29 | EVQLLESGGGLVQPGGSLRLSCAASGFTFNSYAMSWVRQAPGKGLEWVASIKSSGDRVQYADSVKGRFTISRDNSKNTVYLQMNSLRAEDTAIYYCAKPRWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGP**C**VFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |
| GC90LH7-H | 30 | EVQLLESGGGLVQPGGSLRLSCAASGFTFNSYAMSWVRQAPGKGLEWVASIKSSGDRVQYADSVKGRFTISRDNSKNTVYLQMNSLRAEDTAIYYCAKPQWRSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGP**C**VFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |
| GC90LH8-H | 31 | EVQLLESGGGLVQPGGSLRLSCAASGFTFNSYAMSWVRQAPGKGLEWVASIKSSGARVQYADSVKGRFTISRDNSKNTVYLQMNSLRAEDTAIYYCAKPQWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGP**C**VFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |
| GC90LH9-H | 32 | EVQLLESGGGLVQPGGSLRLSCAASGFTFNSYAMSWVRQAPGKGLEWVASIKSSGKRVQYADSVKGRFTISRDNSKNTVYLQMNSLRAEDTAIYYCAKPRWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGP**C**VFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |
| GC90LH10-H | 33 | EVQLLESGGGLVQPGGSLRLSCAASGFTFNSYAMSWVRQAPGKGLEWVASIKSSGARVQYADSVKGRFTISRDNSKNTVYLQMNSLRAEDTAIYYCAKPQWRSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGP**C**VFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |

(continued)

| Antibody | SEQ ID NO: | Amino acid sequence |
|---|---|---|
| GC90LH 11-H | 34 | EVQLLESGGGLVQPGGSLRLSCAASGFTFNSYAMSWVRQAPGKGLE WVASIKSSGKRVQYADSVKGRFTISRDNSKNTVYLQMNSLRAEDTAI YYCAKPQWRSPQRTFEVWGQGTMVTVSSASTKGPSVFPLAPSSKSTS GGTAALGCLVKDYFPEPVTVSWNSGALTSGVHTFPAVLQSSGLYSLSS VVTVPSSSLGTQTYICNVNHKPSNTKVDKRVEPKSCDKTHTCPPCPAP EAAGGPCVFLFPPKPKDTLMISRTPEVTCVVVDVSHEDPEVKFNWYV DGVEVHNAKTKPREEQYNSTYRVVSVLTVLHQDWLNGKEYKCKVS NKALGAPIEKTISKAKGQPREPQVYTLPPSRDELTKNQVSLTCLVKGF YPSDIAVEWESNGQPENNYKTTPPVLDSDGSFFLYSKLTVDKSRWQQ GNVFSCSVLHEALHSHYTQKSLSLSPGK |

Table 2. Amino acid sequences of GPC3 antigen

| Description | SEQ ID NO: | Amino acid sequence |
|---|---|---|
| Human GPC3 protein | 35 | MAGTVRTACLVVAMLLSLDFPGQAQPPPPPPDATCHQVRSFFQR LQPGLKWVPETPVPGSDLQVCLPKGPTCCSRKMEEKYQLTARL NMEQLLQSASMELKFLIIQNAAVFQEAFEIVVRHAKNYTNAMFK NNYPSLTPQAFEFVGEFFTDVSLYILGSDINVDDMVNELFDSLFP VIYTQLMNPGLPDSALDINECLRGARRDLKVFGNFPKLIMTQVS KSLQVTRIFLQALNLGIEVINTTDHLKFSKDCGRMLTRMWYCSY CQGLMMVKPCGGYCNVVMQGCMAGVVEIDKYWREYILSLEE LVNGMYRIYDMENVLLGLFSTIHDSIQYVQKNAGKLTTTIGKLC AHSQQRQYRSAYYPEDLFIDKKVLKVAHVEHEETLSSRRRELIQ KLKSFISFYSALPGYICSHSPVAENDTLCWNGQELVERYSQKAAR NGMKNQFNLHELKMKGPEPVVSQIIDKLKHINQLLRTMSMPKG RVLDKNLDEEGFESGDCGDDEDECIGGSGDGMIKVKNQLRFLA ELAYDLDVDDAPGNSQQATPKDNEISTFHNLGNVHSPLKLLTSM AISVVCFFFLVH |
| GPC3-A peptide | 36 | GDGMIKVKNQLRFLAELAYDLDVDDAPGNSQQATPKDNEISTF HNLGNVHS |
| GPC3-B peptide | 37 | MPKGRVLDKNLDEEGFESGDCGDDEDECIGGSGDGMIKVKNQL RFLAELAYDLD |
| GPC3-B3 | 38 | DKNLDEEGFESG |

Table 3. Numbering system/method definition of CDRs in heavy and light chains variable region of anti-GPC3 antibodies

| Antibody ID | CDR Region | Sequence | Residue position |
|---|---|---|---|
| SEQ ID NOs: 1-2, 6-9 | CDR1 (according to IMGT) | GFTFNSYA | Amino acids 26-33 |
| | CDR2 (according to IMGT) | IKSSGDRV | Amino acids 51-58 |
| | CDR3 (according to IMGT) | AKPQWSSPQRTFEV | Amino acids 97-110 |
| | CDR1 (according to Kabat) | SYAMS | Amino acids 31-35 |
| | CDR2 (according to Kabat) | SIKSSGDRVQYADSVKG | Amino acids 50-66 |
| | CDR3 (according to Kabat) | PQWSSPQRTFEV | Amino acids 99-110 |
| | CDR1 (according to Chothia) | GFTFNSY | Amino acids 26-32 |
| | CDR2 (according to Chothia) | KSSGDR | Amino acids 52-57 |
| | CDR3 (according to Chothia) | PQWSSPQRTFEV | Amino acids 99-110 |
| | CDR1 (according to AbM) | GFTFNSYAMS | Amino acids 26-35 |
| | CDR2 (according to AbM) | SIKSSGDRVQ | Amino acids 50-59 |
| | CDR3 (according to AbM) | PQWSSPQRTFEV | Amino acids 99-110 |
| | CDR1 (according to Contact) | NSYAMS | Amino acids 30-35 |
| | CDR2 (according to Contact) | WVASIKSSGDRVQ | Amino acids 47-59 |
| | CDR3 (according to Contact) | AKPQWSSPQRTFE | Amino acids 97-109 |
| SEQ ID NO: 3 | CDR1 (according to IMGT) | QSISSY | Amino acids 27-32 |
| | CDR2 (according to IMGT) | AA | Amino acids 50-51 |
| | CDR3 (according to IMGT) | QQSYSTPWT | Amino acids 89-97 |
| | CDR1 (according to Kabat) | RASQSISSYLN | Amino acids 24-34 |
| | CDR2 (according to Kabat) | AASSLQS | Amino acids 50-56 |
| | CDR3 (according to Kabat) | QQSYSTPWT | Amino acids 89-97 |
| | CDR1 (according to Chothia) | RASQSISSYLN | Amino acids 24-34 |
| | CDR2 (according to Chothia) | AASSLQS | Amino acids 50-56 |
| | CDR3 (according to Chothia) | QQSYSTPWT | Amino acids 89-97 |
| | CDR1 (according to AbM) | RASQSISSYLN | Amino acids 24-34 |
| | CDR2 (according to AbM) | AASSLQS | Amino acids 50-56 |
| | CDR3 (according to AbM) | QQSYSTPWT | Amino acids 89-97 |
| | CDR1 (according to Contact) | SSYLNWY | Amino acids 30-36 |
| | CDR2 (according to Contact) | LLIYAASSLQ | Amino acids 46-55 |
| | CDR3 (according to Contact) | QQSYSTPW | Amino acids 89-96 |

Through the detailed description and the examples provided below, additional features and advantages of the present invention will become apparent, and should not be construed as limiting. All references, GenBank entries, patents, and published patent applications cited throughout the present invention are hereby expressly incorporated herein by reference in their entirety.

## BRIEF DESCRIPTION OF THE DRAWINGS

FIGS. 1A and 1B illustrate ELISA results of a positive monoclonal antibody screened in Example 1 at a phage level against a GPC3-B peptide.

FIGS. 2 and 3 illustrate affinity of GC90 and mutant monoclonal antibodies thereof for a GPC3 protein.

FIG. 4 illustrates binding of humanized monoclonal antibodies GC90 and hYP7HM to human hepatocellular carcinoma cells HepG2, Hep3B, and Huh-7 at different concentrations.

FIGS. 5A-5D illustrate binding of GC90 and mutant monoclonal antibodies thereof to human hepatocellular carcinoma cells.

FIG. 6 illustrates endocytosis of humanized monoclonal antibodies GC90 and hYP7HM by HepG2 or Hep3B human hepatocellular carcinoma cells at a concentration of 1 nM.

FIG. 7 illustrates cytotoxicity of drug-conjugated forms of GC90 and mutant monoclonal antibodies thereof against human hepatocellular carcinoma cells.

FIGS. 8A and 8B illustrate *in vivo* tumor-inhibitory effects of an antibody-drug conjugate of the present invention.

FIG. 9 illustrates binding strength of GC90 with each overlapping peptide as detected by ELISA, where the underlined amino acid sequences correspond to specific sequences of each overlapping peptide.

FIG. 10 illustrates an amino acid sequence alignment analysis between a human GPC3 protein and a mouse GPC3 protein.

FIG. 11 illustrates the distribution of antigen-recognition regions of GC90 and other reported anti-GPC3 antibodies on a GPC3 protein.

## DETAILED DESCRIPTIONS OF THE EMBODIMENTS

### Example 1

Preparation and isolation of fully human antibody-positive clones targeting a novel GPC3 epitope

Three rounds of biopanning were performed using a natural fully human phage display library and the GPC3-B peptide (corresponding to amino acid residue Met478 to Asp531 of the extracellular domain of GPC3 protein) as an antigen for screening a humanized recombinant antibody library, thereby obtaining a phage library eluate. The neutralized phage library eluate was added to a prepared TG1 bacterial suspension, mixed thoroughly, and incubated statically at 37°C for 45 min to infect TG1 host bacteria. After sufficient infection, the bacterial suspension was serially diluted and plated onto agar plates containing an antibiotic resistance marker, and incubated upside down at 37°C overnight. On the next day, a 96-well sterile deep-well plate was prepared, with 0.5 mL of 2YT dispensed into each well (supplemented with 0.2% w/v glucose and 0.1 mg/mL ampicillin), single bacterial colonies were picked with sterile pipette tips into the corresponding wells and cultured overnight at 37°C with shaking at 220 rpm (16-18 h). On the next day, 0.05-0.1 mL of the overnight cultured monoclonal bacterial suspension was inoculated into a freshly prepared sterile deep-well plate (with 0.5 mL of 2YT medium containing a final concentration of 0.1 mg/mL ampicillin) and cultured until $OD_{600}$ reached approximately 0.6-0.8. A defined proportion of helper phage was added and mixed with shaking to obtain a mixture, the mixture was incubated statically at 37°C for 45 min. Subsequently, 0.25 mL of 2YT medium (containing 0.1 mg/mL ampicillin and kanamycin at a final concentration of 0.05 mg/mL) was added, and the plate was incubated overnight at 30°C with shaking at 220 rpm (16-18 h). The bacterial suspension expressed overnight was centrifuged at 4,000 rpm for 10 min to obtain a phage display expression supernatant, and the supernatant was collected for ELISA detection of GPC3-B peptide and for FACS binding assays for corresponding cells, thereby identifying positive fully human antibody clones.

Indirect ELISA was used to assess the binding capacity of the phage display antibodies in the supernatant to the GPC3-B peptide. An ELISA plate was coated overnight at 4°C with 100 μL/well of GPC3-B peptide at 4 μg/mL in CBS coating buffer. The plate was washed with PBST (containing 0.05% Tween) and blocked with 300 μL/well PBS containing 3% skim milk at 37°C for 1 h. The blocking solution was then discarded, 50 μL of serially diluted phage expression supernatants and 50 μL of 0.05% PBST were added to each well, a negative control (IPI: ipilimumab) was added, and the plate was then incubated at room temperature for 2 h. The plate was washed three times with 0.05% PBST and incubated with 100 μL/well of goat anti-M13 phage antibody conjugated with horseradish peroxidase (Sino Biological) at room temperature for 45 min. The plate was washed six times with 0.05% PBST, and TMB substrate solution (GenScript) was added and incubated at room temperature in the dark for 10 min. The reaction was stopped by adding 50 μL of 1 M HCl stop solution (Sigma). Absorbance was measured at 450 nm using a microplate reader. The binding assay results are shown in FIGS. 1A-1B, demonstrating that multiple positive monoclonal antibodies exhibited strong binding to the GPC3-B peptide at a phage level.

Further, ELISA was conducted to identify whether the selected positive monoclonal antibodies recognized the GPC3-A peptide. The specific procedure was as follows: GPC3-A peptide and GPC3-B peptide (6 μg/mL) were coated into the 96-well plate with 100 μL/well and incubated overnight at 4°C. The plate was then blocked at 37°C for 2 h with PBST (containing 0.05% Tween-20) containing 1% BSA (200 μL/well), and was then washed three times with PBST. Positive monoclonal antibodies GC008, GC010, GC011, GC025, GC035, GC037, GC053, GC067, GC139, GC147, GC90, as well as the control antibody hYP7HM, were serially diluted in PBST containing 1% BSA and added sequentially to the 96-well

plate (100μL/well), with working concentrations of 30 nM and 15 nM, with a total of 8 additional points generated by 4-fold serial dilutions starting from 15 nM. The plate was incubated at 37°C for 1 h and washed three times with PBST. Then, anti-human IgG-Fc-HRP (Sigma, 1: 30,000 dilution) was added at 100 μL/well and incubated at 37°C for 1 h, the plate was then washed three times with PBST, 50 μL of TMB (SURMOPICS) was added, and the reaction was stopped with 1M $H_2SO_4$. Absorbance at 450 nm was measured with a microplate reader. The results are shown in Table 4. The positive monoclonal antibodies identified in this Example specifically bound only to the GPC3-B peptide, while the control antibody hYP7HM bound exclusively to the GPC3-A peptide (consistent with the patent literature), indicating that the positive monoclonal antibodies screened in this Example have a different antigen recognition site compared to the control anti-GPC3 antibody hYP7HM.

Table 4. Binding of anti-GPC3 antibodies to GPC3-A peptide and GPC3-B peptide

| Antigen \ Antibody | GC008 | GC010 | GC011 | GC025 | GC035 | GC037 | GC053 |
|---|---|---|---|---|---|---|---|
| GPC3-A peptide | - | - | - | - | - | - | - |
| GPC3-B peptide | ++++ | ++++ | ++++ | ++++ | ++++ | ++++ | ++++ |

| Antigen \ Antibody | GC067 | GC139 | GC147 | GC90 | hYP7HM | | |
|---|---|---|---|---|---|---|---|
| GPC3-A peptide | - | - | - | - | ++++ | | |
| GPC3-B peptide | ++++ | ++++ | ++++ | ++++ | - | | |

**[0149]** Note: "–" indicates no binding; "++++" indicates that an $OD_{450}$ value through ELISA is greater than 1.5.

The positive clones selected in this Example were sequenced, and the amino acid sequences of the heavy chain variable region and light chain variable region are shown in Table 5.

Table 5. Light chain and heavy chain variable region sequences of anti-GPC3 antibody

| Antibody | VH/VL | SEQ ID NO: | Amino acid sequence |
|---|---|---|---|
| GC008 | VL | 39 | DIQLTQSPSSLSASVGDRVTITCQASQDIDNSVTWYQQRPGKAPSLLISDASILESGVPSRFSGRGSGTHFTLSITSLQPEDIATYYCQQYENLPITFGQGTRLEIK |
| | VH | 40 | EVQLLESGGGLVQPGGSMRLSCAASEIDFSVYALGWSRQAPGKQRELVATITTSSTTTYADSVKGRFTISRDNAKNTVYLQMNSLKPEDTAVYYCHTLRRTGSGIVPGRIWGQGTPVTVSS |
| GC010 | VL | 41 | DIQMIQSPSSLSASVGDRVTITCRASQSISSYLNWYQQKPGKAPKLLIYAASSLQSGVPSRFSGSGSGTDFTLTISSLQPEDFATYYCQQGYSTPFTLGPGTKVDIK |
| | VH | 42 | EVQLLESGGGVVQPGRSLRLSCAASGFTFSSYAMHWVRQAPGKGLEWVAVISYDGSNKYYADSVKGRFTISRDNSKNTLYLQMNSLRAEDTAVYYCARDESRRKFDYWGQGTTVTVSS |
| GC011 | VL | 43 | NIQMTQSPSSLSASVGDRVTITCRASQSITRYLNWYQQKPGKGPKLLIYAASRLQSGVPSRFSGSGSGTDFTLTISSLQPEDFATYYCQQSHSIPRTFGQGTKLEIK |
| | VH | 44 | EVQLLESGGGVVQPGRSLRLSCAASGFTFSSYGMHWVRQAPGKGLEWVAVISYDGSNKYYADSVKGRFTISRDNSKNTLYLQMNSLRAEDTAVYYCAKNPSLDYWGQGTTVTVSS |

(continued)

| Antibody | VH/VL | SEQ ID NO: | Amino acid sequence |
|---|---|---|---|
| GC025 | VL | 45 | DIQLTQSPSSLSASVGDRVTITCRASQSISSYLSWYQQKPGKAPKLLIYGASSLQSGVPSRFSGSGSGTDFTLTISSLQPEDFATYSCQQSYSTQFTFGPGTKVDIK |
| | VH | 46 | EVQLLESGGGVVQPGRSLRLSCAASGFTFSSYAMHWVRQAPGKGLEWVAVISYDGSNKYYADSVKGRFTISRDNSKNTLYLQMNSLRAEDTAVYYCARRQRAAIDYWGQGTTVTVSS |
| GC035 | VL | 47 | DIQLTQSPSSLSASVGDRVTITCRAGQTINNYLHWYQQKPGKAPKLLIFAASSLQSGVPSRFSGSGSGTDFTLTISSLQPEDFATYYCQQSYSPPLTFGGGTRVEIK |
| | VH | 48 | EVQLLESGGGVVRPGRSLRLSCAASGFTFSSYAMHWVRQAPGKGLEWVAVISYDGSNKYYADSVKGRFTISRDNSKNTLYLQMNSLRAEDTAVYYCARRKSGSSFDYWGQGTPVTVSS |
| GC037 | VL | 49 | AIRLTQFPSSLSASVGDRVTITCRARQSIDNYLNWYQEKPGKAPKLLIYAASSLQSGVPSRFSGSGSGTEFTLTISSLQPDDFAAYYCQQSYDSPPTFGQGTKVEIK (SEQ ID NO: ) |
| | VH | 50 | EVQLLESGGGLVQPGGSLRLSCAASGFTFSSYAMHWVRQAPGKGLEYVSAISSNGGSTYYANSVKGRFTISRHPSKNILFLEMNSLRPDDTAVYYCVRGWIRPPPGSFDLWGQGTPVTVSS |
| GC053 | VL | 51 | NIQMTQSPSSLSAFVGDRVTITCRASQSISRYLNWYQHKPGQAPKLLMYAASTLQDEVPSRFSGSGSGTYFTLTISSLEPEDFATYYCQQSYSTPPTFGGGTKVEIK |
| | VH | 52 | EVQLLESGGGLVQPGGSLRLSCAASGFTFSSYAMHWVRQAPGKGLEYVSAISSNGGSTYYANSVKGRFTISRDNSKNTLYLQMGSLRPDDTAVYYCVRGWIRPPPGSFDLWGQGTTVTVSS |
| GC067 | VL | 53 | NIQMTQSPSSLSASVGDRVTITCRASQSISSYLNWYQQKPGKAPKLLIYAASSLQSGVPSRFSGSGSGTDFTLTISSLQPEDFATYYCQQSYSTPLTFGGGTKVEIK |
| | VH | 54 | EVQLLESGGGVVQPGRSLRLSCAASGFTFSSYAMHWVRQAPGKGLEWVAVISYDGSNKYYADSVKGRFTISRDNSKNTLYLQMNSLRAEDTAVYYCARRLRGSGMDVWGQGTPVTVSS |
| GC139 | VL | 55 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPPTFGPGTKVDIK |
| | VH | 56 | EVQLVESGGGLIQPGGSLRLSCAASGFTFSSYAMHWVRQAPGKGLEYVSAISSNGGSTYYADSVKGRFTISRDNSKNTLYLQMSSLRPDDTAVYYCVRGWIRPPPGSFDLWGQGTTVTVSS |
| GC147 | VL | 57 | AIRLTQSPSSLSASVGDRVSITCRASQSISTFLNWYQQKPGKAPNLLIYSASTLQSGVPSRFSGSGSGTDFTLTISSLQPEDFATYYCQQSYSSPPTFGQGTKLEIK |
| | VH | 58 | EVQLVESGGGLVQPGGSLRLSCAASGFTFSSYAMHWVRQAPGKGLEYVSAISSNGGSTYYANSVKGRFTISRDNSKNTLYLQMGSLRAEDMAVYYCVRGWIRPPPGSFDLWGQGTPVTVSS |

(continued)

| Antibody | VH/VL | SEQ ID NO: | Amino acid sequence |
|---|---|---|---|
| GC90 | VL | 3 | DIQLTQSPSSLSASVGDRVTITCRASQSISSYLNWYQQKPGKAPK LLIYAASSLQSGVPSRFSGSGSGTDFTLTISSLQPEDFATYYCQQSY STPWTFGQGTKVEIK |
| | VH | 1 | QVQLLESGGGLVQPGGSLRVSCAASGFTFNSYAMSWVRQAPGK GLEWVASIKSSGDRVQYADSVKGRFTISRDNSKNTVYLEMNSLR AEDTAIYYCAKPQWSSPQRTFEVWGQGTPVTVSS |

**Example 2**

Preparation of humanized monoclonal antibodies

2.1 Vector construction

First, the nucleotide sequence encoding the human IgG1 heavy chain constant region (IgG1-CH) was coupled with a secretion signal peptide (SP) coding sequence at an upstream region and a stop codon (TAG) at a downstream region. The gene was synthesized and cloned into a pcDNA3.1 vector (Sangon Biotech (Shanghai) Co., Ltd.), and the fragment was then inserted downstream of the CMV promoter in the pCHOGUN vector using an In-fusion cloning method. Specifically, the synthesized gene fragment and the pCHOGUN vector plasmid fragment were amplified using primers with specific insertion sites and a high-fidelity PCR enzyme (HiFi PCR Premix, TAKARA), respectively. After gel recovery, the IgG1 heavy chain fragment and linearized vector were ligated (using In-fusion Snap Assembly Master Mix, TAKARA), generating a heavy chain constant region expression vector pCHOGUN-IgG1. Following the above implementation method, the sequence encoding the human immunoglobulin κ light chain constant region (IgG-CK) was modified by adding a signal peptide (SP) coding sequence to the upstream region and the stop codon (TAG) to the downstream region. After gene synthesis, the sequence was inserted downstream of the CMV promoter in the pCHOGUN vector to generate a light chain constant region vector pCHOGUN-CK.

For constructing an expression vector of each humanized monoclonal antibody, coding sequences of the heavy chain variable region (VH) and light chain variable region (VL) of GC90 and its optimized mutants were synthesized and inserted between the SP sequence and the constant region in the pCHOGUN-IgG1 and pCHOGUN-CK vectors using the above in-fusion cloning method, thereby generating the heavy chain and light chain expression vectors of the monoclonal antibodies. The specific antibody sequences are shown in Table 6.

Table 6. Heavy chain and light chain sequences of humanized monoclonal antibodies

| | | |
|---|---|---|
| GC90 | L chain SEQ ID NO: 10 | DIQLTQSPSSLSASVGDRVTITCRASQSISSYLNWYQQKPGKAPKLLIYAAS SLQSGVPSRFSGSGSGTDFTLTISSLQPEDFATYYCQQSYSTPWTFGQGTKV EIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSP VTKSFNRGEC |
| | H chain SEQ ID NO: 11 | QVQLLESGGGLVQPGGSLRVSCAASGFTFNSYAMSWVRQAPGKGLEWVA SIKSSGDRVQYADSVKGRFTISRDNSKNTVYLEMNSLRAEDTAIYYCAKP QWSSPQRTFEVWGQGTPVTVSSASTKGPSVFPLAPSSKSTSGGTAALGCL VKDYFPEPVTVSWNSGALTSGVHTFPAVLQSSGLYSLSSVVTVPSSSLGTQ TYICNVNHKPSNTKVDKRVEPKSCDKTHTCPPCPAPELLGGPSVFLFPPKP KDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPV LDSDGSFFLYSKLTVDKSRWQQGNVFSCSVMHEALHNHYTQKSLSLSPG K |

(continued)

| | | |
|---|---|---|
| GC90-4mu | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 12 | QVQLLESGGGLVQPGGSLRVSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGPCVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| GC90-6mu | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 13 | QVQLLESGGGLVQPGGSLRVSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGPCVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |
| GC90LH1 | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 14 | EVQLLESGGGLVQPGGSLRLSCAASGFTFNSYAMSWVRQAPGKGLEWVASIKSSGDRVQYADSVKGRFTISRDNSKNTVYLEMNSLRAEDTAIYYCAKPQWSSPQRTFEVWGQGTPVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |

(continued)

| | | |
|---|---|---|
| GC90LH2 | L chain SEQ ID NO: 10 | DIQLTQSPSSLSASVGDRVTITCRASQSISSYLNWYQQKPGKAPKLLIYAAS<br>SLQSGVPSRFSGSGSGTDFTLTISSLQPEDFATYYCQQSYSTPWTFGQGTKV<br>EIKRTVAAPSVFIFPPSDEQLKSGTASVVCLLNNFYPREAKVQWKVDNAL<br>QSGNSQESVTEQDSKDSTYSLSSTLTLSKADYEKHKVYACEVTHQGLSSP<br>VTKSFNRGEC |
| | H chain SEQ ID NO: 15 | QVQLLESGGGLVQPGGSLRVSCAASGFTFNSYAMSWVRQAPGKGLEWVA<br>SIKSSGDRVQYADSVKGRFTISRDNSKNTVYLQMNSLRAEDTAIYYCAKP<br>QWSSPQRTFEVWGQGTPVTVSSASTKGPSVFPLAPSSKSTSGGTAALGCL<br>VKDYFPEPVTVSWNSGALTSGVHTFPAVLQSSGLYSLSSVVTVPSSSLGTQ<br>TYICNVNHKPSNTKVDKRVEPKSCDKTHTCPPCPAPELLGGPSVFLFPPKP<br>KDTLMISRTPEVTCVVVDVSHEDPEVKFNWYVDGVEVHNAKTKPREEQY<br>NSTYRVVSVLTVLHQDWLNGKEYKCKVSNKALPAPIEKTISKAKGQPREP<br>QVYTLPPSRDELTKNQVSLTCLVKGFYPSDIAVEWESNGQPENNYKTTPPV<br>LDSDGSFFLYSKLTVDKSRWQQGNVFSCSVMHEALHNHYTQKSLSLSPG<br>K<br>DIQLTQSPSSLSASVGDRVTITCRASQSISSYLNWYQQKPGKAPKLLIYAAS<br>SLQSGVPSRFSGSGSGTDFTLTISSLQPEDFATYYCQQSYSTPWTFGQGTKV |
| GC90LH3 | L chain SEQ ID NO: 10 | DIQLTQSPSSLSASVGDRVTITCRASQSISSYLNWYQQKPGKAPKLLIYAAS<br>SLQSGVPSRFSGSGSGTDFTLTISSLQPEDFATYYCQQSYSTPWTFGQGTKV<br>EIKRTVAAPSVFIFPPSDEQLKSGTASVVCLLNNFYPREAKVQWKVDNAL<br>QSGNSQESVTEQDSKDSTYSLSSTLTLSKADYEKHKVYACEVTHQGLSSP<br>VTKSFNRGEC |
| | H chain SEQ ID NO: 16 | QVQLLESGGGLVQPGGSLRVSCAASGFTFNSYAMSWVRQAPGKGLEWVA<br>SIKSSGDRVQYADSVKGRFTISRDNSKNTVYLEMNSLRAEDTAIYYCAKP<br>QWSSPQRTFEVWGQGTMVTVSSASTKGPSVFPLAPSSKSTSGGTAALGCL<br>VKDYFPEPVTVSWNSGALTSGVHTFPAVLQSSGLYSLSSVVTVPSSSLGTQ<br>TYICNVNHKPSNTKVDKRVEPKSCDKTHTCPPCPAPELLGGPSVFLFPPKP<br>KDTLMISRTPEVTCVVVDVSHEDPEVKFNWYVDGVEVHNAKTKPREEQY<br>NSTYRVVSVLTVLHQDWLNGKEYKCKVSNKALPAPIEKTISKAKGQPREP<br>QVYTLPPSRDELTKNQVSLTCLVKGFYPSDIAVEWESNGQPENNYKTTPPV<br>LDSDGSFFLYSKLTVDKSRWQQGNVFSCSVMHEALHNHYTQKSLSLSPG<br>K |
| GC90LH4 | L chain SEQ ID NO: 10 | DIQLTQSPSSLSASVGDRVTITCRASQSISSYLNWYQQKPGKAPKLLIYAAS<br>SLQSGVPSRFSGSGSGTDFTLTISSLQPEDFATYYCQQSYSTPWTFGQGTKV<br>EIKRTVAAPSVFIFPPSDEQLKSGTASVVCLLNNFYPREAKVQWKVDNAL<br>QSGNSQESVTEQDSKDSTYSLSSTLTLSKADYEKHKVYACEVTHQGLSSP<br>VTKSFNRGEC |
| | H chain SEQ ID NO: 17 | EVQLLESGGGLVQPGGSLRLSCAASGFTFNSYAMSWVRQAPGKGLEWVA<br>SIKSSGDRVQYADSVKGRFTISRDNSKNTVYLQMNSLRAEDTAIYYCAKP<br>QWSSPQRTFEVWGQGTPVTVSSASTKGPSVFPLAPSSKSTSGGTAALGCL<br>VKDYFPEPVTVSWNSGALTSGVHTFPAVLQSSGLYSLSSVVTVPSSSLGTQ<br>TYICNVNHKPSNTKVDKRVEPKSCDKTHTCPPCPAPELLGGPSVFLFPPKP<br>KDTLMISRTPEVTCVVVDVSHEDPEVKFNWYVDGVEVHNAKTKPREEQY<br>NSTYRVVSVLTVLHQDWLNGKEYKCKVSNKALPAPIEKTISKAKGQPREP<br>QVYTLPPSRDELTKNQVSLTCLVKGFYPSDIAVEWESNGQPENNYKTTPPV<br>LDSDGSFFLYSKLTVDKSRWQQGNVFSCSVMHEALHNHYTQKSLSLSPG<br>K |

(continued)

| | | |
|---|---|---|
| GC90LH5 | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 18 | EVQLLESGGGLVQPGGSLRLSCAASGFTFNSYAMSWVRQAPGKGLEWVASIKSSGDRVQYADSVKGRFTISRDNSKNTVYLQMNSLRAEDTAIYYCAKPQWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| GC90LH6 | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 29 | EVQLLESGGGLVQPGGSLRLSCAASGFTFNSYAMSWVRQAPGKGLEWVASIKSSGDRVQYADSVKGRFTISRDNSKNTVYLQMNSLRAEDTAIYYCAKPRWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGPCVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |
| GC90LH7 | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 30 | EVQLLESGGGLVQPGGSLRLSCAASGFTFNSYAMSWVRQAPGKGLEWVASIKSSGDRVQYADSVKGRFTISRDNSKNTVYLQMNSLRAEDTAIYYCAKPQWRSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGPCVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |

(continued)

| | | |
|---|---|---|
| GC90LH8 | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 31 | EVQLLESGGGLVQPGGSLRLSCAASGFTFNSYAMSWVRQAPGKGLEWVASIKSSGARVQYADSVKGRFTISRDNSKNTVYLQMNSLRAEDTAIYYCAKPQWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGPCVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |
| GC90LH9 | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 32 | EVQLLESGGGLVQPGGSLRLSCAASGFTFNSYAMSWVRQAPGKGLEWVASIKSSGKRVQYADSVKGRFTISRDNSKNTVYLQMNSLRAEDTAIYYCAKPRWSSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGPCVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |
| GC90LH10 | L chain SEQ ID NO: 10 | DIQLTQSPSSLSASVGDRVTITCRASQSISSYLNWYQQKPGKAPKLLIYAASSLQSGVPSRFSGSGSGTDFTLTISSLQPEDFATYYCQQSYSTPWTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| | H chain SEQ ID NO: 33 | EVQLLESGGGLVQPGGSLRLSCAASGFTFNSYAMSWVRQAPGKGLEWVASIKSSGARVQYADSVKGRFTISRDNSKNTVYLQMNSLRAEDTAIYYCAKPQWRSPQRTFEVWGQGTMVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPEAAGGPCVFLFPPKPKDTLMISRTPEVTCVVVDVSHEDPEVKFNWYVDGVEVHNAKTKPREEQYNSTYRVVSVLTVLHQDWLNGKEYKCKVSNKALGAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVLHEALHSHYTQKSLSLSPGK |

(continued)

| | | |
|---|---|---|
| GC90LH11 | L chain SEQ ID NO: 10 | DIQLTQSPSSLSASVGDRVTITCRASQSISSYLNWYQQKPGKAPKLLIYAAS SLQSGVPSRFSGSGSGTDFTLTISSLQPEDFATYYCQQSYSTPWTFGQGTKV EIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSP VTKSFNRGEC |
| | H chain SEQ ID NO: 34 | EVQLLESGGGLVQPGGSLRLSCAASGFTFNSYAMSWVRQAPGKGLEWVA SIKSSGKRVQYADSVKGRFTISRDNSKNTVYLQMNSLRAEDTAIYYCAKP QWRSPQRTFEVWGQGTMVTVSSASTKGPSVFPLAPSSKSTSGGTAALGCL VKDYFPEPVTVSWNSGALTSGVHTFPAVLQSSGLYSLSSVVTVPSSSLGTQ TYICNVNHKPSNTKVDKRVEPKSCDKTHTCPPCPAPEAAGGPCVFLFPPKP KDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREP QVYTLPPSRDELTKNQVSLTCLVKGFYPSDIAVEWESNGQPENNYKTTPPV LDSDGSFFLYSKLTVDKSRWQQGNVFSCSVLHEALHSHYTQKSLSLSPGK |

2.2 Cell transfection and expression

Transfection was performed according to the TransIT-PRO® Transfection Reagent (Mirus) instructions. As described below, ExpiCHO-S cells (Thermo) were cultured in a complete medium (CHOgro® High Yield Expression System, containing 30 mL/L of a 10% Poloxamer 188 solution and 20 mL/L of a 200 mM L-glutamine, Mirus). The cells were subcultured for 24 h before transfection and diluted to a density of $2\times10^6$ cells/mL to ensure that a density of the cells reached $4\times10^6$ cells/mL the next day. Heavy chain and light chain plasmids (25 μg each) were mixed at a ratio of 1:1 to 12.5 mL of a complete medium and mixed well, 50 μL of TransIT-PRO® Transfection Reagent (Mirus) was added, mixed gently by inversion, and allowed to stand for 4 min to obtain a mixture; and the mixture was then added dropwise to 50 mL of diluted cells while gently shaking, and 1 mL of CHOgro-titer Enhancer (Mirus) was then added dropwise. The cells were immediately placed in a 32°C, 5% $CO_2$ incubator, 5% of Efficient Feed C + AGT Supplement (Thermo) was added every other day, and culture was continued for a total of 7 days.

2.3 Antibody purification

After culturing in a shake flash for 7 d, a cell supernatant was collected, centrifuged at 4,000 rpm for 20 min, and filtered through a 0.22-μm membrane (Milipore), and antibodies were purified by Protein A affinity chromatography. Briefly, a HiTrap MabSelect suRe prepacked column (Cytiva) was equilibrated with 5-10 column volumes of 20 mM PB + 0.15 M NaCl buffer, an AKTA Avant 150 chromatography system (Cytiva) was used, the filtered supernatant was loaded onto the column, which was then washed with 3 column volumes of 20 mM PB + 0.15 M NaCl buffer followed by 1 column volume of 20 mM PB + 1 M NaCl buffer, and subsequently washed with 20 mM PB until a stable baseline was achieved. Finally, the antibody was eluted with 20 mM citric acid (adjusted to pH 3.0 with 20 mM of sodium citrate), elution peaks ranging from 200 mAu to 200 mAu were collected. The eluted antibody was immediately neutralized with a neutralization buffer (1 M Tris-HCl, pH 9.0) and stored in a 1.5 mL tube for freezing at -80°C for later use.

**Example 3**

ELISA-based affinity assay of humanized monoclonal antibodies

The relative binding activity of the antibodies to human GPC3 protein was determined using an ELISA assay. The specific procedure was as follows: recombinant human GPC3 protein (1 μg/mL) was coated onto a 96-well plate at 100 μL/well and incubated overnight at 4°C. The plate was then blocked at 37°C for 2 h with PBST (containing 0.05% Tween-20) containing 1% BSA (200 μL/well), and was then washed three times with PBST. Each humanized monoclonal antibody was serially diluted in PBST containing 1% BSA and added sequentially to the 96-well plate (100 μL/well). Working concentrations of the humanized monoclonal antibodies GC90, GC90-4mu, GC90LH1, GC90LH2, GC90LH3, GC90LH4 and GC90LH5 were 15 nM and 3.75 nM, with a total of 8 additional points generated by 4-fold serial dilutions starting from 3.75 nM. The plate was incubated at 37°C for 1 h and washed three times with PBST. Then, anti-human IgG-Fc-HRP (Sigma, 1: 30,000 dilution) was added at 100 μL/well and incubated at 37°C for 1 h, the plate was then washed three times again with PBST, 50 μL of TMB (SURMOPICS) was added for reaction, and the reaction was stopped with 1M $H_2SO_4$. Absorbance at 450 nm-570 nm was measured with a microplate reader. Based on the results shown in FIG. 2, the

GC90 monoclonal antibody and a variant thereof of the present invention exhibit excellent binding affinity for GPC3 protein.

**Example 4**

ELISA detection of affinity-matured antibody molecules

The relative binding activity of the antibodies to human GPC3 protein was determined using an ELISA assay. The specific procedure was as follows: recombinant human GPC3 protein (1 μg/mL) was coated onto a 96-well plate at 100 μL/well and incubated overnight at 4°C. The plate was then blocked at 37°C for 2 h with PBST (containing 0.05% Tween-20) containing 1% BSA (200μL/well), and was then washed three times with PBST. Each humanized monoclonal antibody was serially diluted in PBST containing 1% BSA and added sequentially to the 96-well plate (100 μL/well). Working concentrations of the humanized monoclonal antibodies GC90-4mu, GC90LH6, GC90LH7 and GC90LH8 were 10,000 ng/μL, and 3,333.33 ng/μL, with a total of 10 additional points generated by 3-fold serial dilutions starting from 1,111.11 ng/μL; and working concentrations of GC90LH8 and GC90LH10 were 10,000 ng/μL and 2,500ng/μL, with a total of 8 additional points generated by 4-fold serial dilutions starting from 625 ng/μL. The plate was incubated at 37°C for 1 h and washed three times with PBST. Then, anti-human IgG-Fc-HRP (Sigma, 1: 30,000 dilution) was added at 100 μL/well and incubated at 37°C for 1 h, the plate was then washed three times with PBST, 50 μL of TMB (SURMOPICS) was added, and the reaction was stopped with 1M $H_2SO_4$. Absorbance at 450 nm-570 nm was measured with a microplate reader. Based on the results shown in FIG. 3, the GC90 monoclonal antibody and a variant thereof of the present invention exhibit excellent binding affinity for GPC3 protein.

**Example 5**

Flow cytometry detection of binding of humanized monoclonal antibodies to tumor cells

Flow cytometry was used to determine the affinity of each anti-GPC3 antibody for HepG2, Hep3B, and Huh7 cells. The specific procedure was as follows: each humanized monoclonal antibody was serially diluted using FACS Buffer (PBS + 5% FBS) and added sequentially to a 96-well U-shaped plate. Working concentrations of the antibodies were 100 nM and 25 nM, with a total of 6 additional points generated by 4-fold serial dilutions starting from 6.25 nM. HepG2, Hep3B, and Huh7 cells were digested with trypsin, centrifuged at 1,000 rpm for 5 min, and a supernatant was discarded. The cells were resuspended in FACS buffer at a concentration of $2 \times 10^6$ cells/mL, and 50μL of the cell suspension was added to each well of a 96-well U-bottom plate. Subsequently, 50 μL of each diluted antibody was added to each well, mixed gently, and incubated on ice for 90 min. After centrifugation at 3,500 rpm for 3 min at 4°C, a supernatant was discarded. The cells were then resuspended in 250 μL of pre-chilled FACS buffer and washed twice by centrifugation. 100 μL of diluted PE anti-human IgG FC fluorescent secondary antibody (BioLegend; prepared at 0.5 μL per $1 \times 10^5$ cells) was added to each well and incubated on ice in the dark for 30 min. The cells were washed twice with the supernatant discarded, and finally resuspended in 200 μL of FACS Buffer. A mean fluorescence intensity (MFI) was measured using an Attune NxT flow cytometer (Thermo), and data were processed using GraphPad Prism software. As shown in FIG. 4, the monoclonal antibodies of the present invention exhibit excellent affinity for HepG2, Hep3B, and Huh-7 cells.

**Example 6**

Flow cytometry detection of binding of humanized monoclonal antibodies to tumor cells

Flow cytometry was used to determine the affinity of each anti-GPC3 antibody for HepG2 or Hep3B cells. The specific procedure was as follows: the humanized monoclonal antibodies GC90-4mu, GC90, GC90LH1, GC90LH2, GC90LH3, GC90LH4 and GC90LH5 were serially diluted using FACS Buffer (PBS + 5% FBS) and added sequentially to 96-well U-shaped plates, and working concentrations of the antibodies were 100 nM and 25 nM, with a total of 8 additional points generated by 4-fold serial dilutions starting from 6.25 nM. HepG2 and Hep3B cells were digested with trypsin, centrifuged at 1,000 rpm for 5 min, and a supernatant was discarded. The cells were resuspended in FACS buffer at a concentration of $2 \times 10^6$ cells/mL, and 50 μL of the cell suspension was added to each well of a 96-well U-bottom plate. Subsequently, 50 μL of each diluted antibody was added to each well, mixed gently, and incubated on ice for 90 min. After centrifugation at 3,500 rpm for 3 min at 4°C, a supernatant was discarded. The cells were then resuspended in 250 μL of pre-chilled FACS buffer and washed twice by centrifugation. 100 μL of diluted PE anti-human IgG FC fluorescent secondary antibody (BioLegend; prepared at 0.5 μL per $1 \times 10^5$ cells) was added to each well and incubated on ice in the dark for 30 min. The cells were washed twice with the supernatant discarded, and finally resuspended in 200 μL of FACS Buffer. An MFI was measured using an Attune NxT flow cytometer (Thermo), and data were processed using GraphPad Prism software. The results are shown in FIGS. 5A-5B, demonstrating that the monoclonal antibodies of the present

invention exhibit excellent affinity for HepG2 or Hep3B cells.

**Example 7**

Flow cytometry detection of binding of affinity-matured monoclonal antibodies to tumor cells

Flow cytometry was used to determine the affinity of each anti-GPC3 antibody for HepG2 cells. The specific procedure was as follows: the humanized monoclonal antibodies IgG1, GC90-4mu, GC90LH6, GC90LH7, GC90LH8, GC90LH9, GC90LH10 and GC90LH11 were serially diluted using FACS Buffer (PBS + 5% FBS) and added sequentially to 96-well U-shaped plates, and working concentrations of the antibodies were 100 nM and25 Nm, and with a total of 8-9 additional points generated by 4-fold serial dilutions starting from 6.25 nM. HepG2 cells were digested with trypsin, centrifuged at 1,000 rpm for 5 min, and a supernatant was discarded. The cells were resuspended in FACS buffer at a concentration of $2 \times 10^6$ cells/mL, and 50 μL of the cell suspension was added to each well of a 96-well U-bottom plate. Subsequently, 50 μL of each diluted antibody was added to each well, mixed gently, and incubated on ice for 90 min. After centrifugation at 3,500 rpm for 3 min at 4°C, a supernatant was discarded. The cells were then resuspended in 250 μL of pre-chilled FACS buffer and washed twice by centrifugation. 100 μL of diluted PE anti-human IgG FC fluorescent secondary antibody (BioLegend, 0.5μL/$1\times10^5$ cells) was added to each well and incubated on ice in the dark for 30 min. The cells were washed twice with the supernatant discarded, and finally resuspended in 200 μL of FACS Buffer. A mean fluorescence intensity (MFI) was measured using an Attune NxT flow cytometer (Thermo), and data were processed using GraphPad Prism software. As shown in FIGS. 5C-5D, the monoclonal antibodies of the present invention exhibit excellent affinity for HepG2 cells.

**Example 8**

Flow cytometry detection of endocytosis efficiency of humanized monoclonal antibodies to tumor cells

Flow cytometry was used to determine the endocytosis efficiency of each anti-GPC3 antibody for HepG2 and Hep3B cells. The specific procedure was as follows: GC90 and hYP7HM monoclonal antibodies were diluted to a final experimental concentration of 1 nM using FACS buffer, and each antibody solution was added to 50 μL per well of a 96-well U-shaped plate. Antibody binding to cells was performed according to the method described in Example 5. After washing away unbound antibodies, the cells were incubated at 37°C for 0, 1, 2, or 4 h, respectively. Immediately after incubation, 100 μL of pre-chilled FACS buffer was added to terminate endocytosis. The cells were then centrifuged at 3,500 rpm for 3 min, and the supernatant was discarded. Secondary antibody was prepared according to the method described in Example 5. Cells were resuspended in 100 μL of the secondary antibody per well and incubated on ice for 30 min. The cells were washed twice by centrifugation, and finally resuspended in 200 μL of FACS buffer, and an MFI was measured, and data were processed using GraphPad Prism software. The endocytosis efficiency of the humanized antibodies was calculated using the following formula: ((1 - MFI value of antibody at 37°C/ MFI value of antibody at 4°C) $\times$ 100%). The results are shown in FIG. 6. As shown in FIG. 6, the humanized antibodies of the present invention exhibit high endocytosis efficiency in both HepG2 and Hep3B cells.

**Example 9**

Preparation of antibody-drug conjugate

9.1 Site-specific conjugation

The heavy chain residue 239 of the antibody was mutated to cysteine (S239C, EU numbering, corresponding to a position 243 of the GC90-4mu heavy chain) to enable site-specific conjugation of the drug via a linker at this cysteine residue. Specifically, the monoclonal antibody solution was replaced with 20 mM PBS buffer (pH 7.2) and adjusted to a concentration of approximately 5 mg/mL. 250 mM of EDTA solution was added at a volume ratio of 50: 1 (antibody : EDTA) and mixed thoroughly to obtain a mixture. Subsequently, depending on the combination of different monoclonal antibodies with linker-payload (LP), 1-12 times of tris(2-carboxyethyl)phosphine hydrochloride (TCEP) was added to the mixture in an excess molar ratio (relative to the antibody) and mixed thoroughly, and incubated at room temperature (25°C) for 3 h for reduction reaction. An appropriate amount of DMSO was then added to the above reaction mixture, LP drug (5 mM or 10 mM stock solution in DMSO) in an excess molar ratio (relative to the antibody) of 6-12 times was added to the mixture, ensuring that a final DMSO content in the reaction system did not exceed 15% (v/v). The mixture was mixed thoroughly and incubated at room temperature for 1.5 h. N-acetyl-L-cysteine (NAC) solution was then added, and the reaction was terminated by standing at room temperature for 10 min.

The reaction mixture was desalted by ultrafiltration to obtain a reaction solution, the reaction solution was transferred to a 10 KD ultrafiltration tube (Millipore), a PBS buffer (pH 6.0) was added, and the mixture was concentrated by centrifugation at 3,500 g to a desired volume, and a PBS was added and concentrated again for a total of 5 cycles to obtain a product. The product was filtered through a 0.22 μm filter membrane (Millipore) and stored at -80°C.

After the conjugation reaction, site-directed conjugation products of IgG1-PBD, GC90-4mu-PBD, GC90LH8-PBD, GC90LH9-PBD, GC90LH10-PBD, GC90LH11-PBD, as well as IgG1-DUBA, GC90-4mu-DUBA, and hYP7HM-DUBA were obtained. A purity of the ADC products was analyzed by size-exclusion chromatography (SEC), and a drug-to-antibody ratio (DAR) and unconjugated antibody fraction were analyzed by hydrophobic interaction chromatography (HIC).

Table 7. ADC product detection and analysis

| S/N | ADC | DAR (HIC) |
|---|---|---|
| 1 | GC90-4mu-PBD | 1.8 |
| 2 | IgG1-PBD | 1.89 |
| 3 | GC90LH8-PBD | 1.9 |
| 4 | GC90LH9-PBD | 1.85 |
| 5 | GC90LH10-PBD | 1.92 |
| 6 | GC90LH11-PBD | 1.83 |
| 7 | IgG1-DUBA | 1.77 |
| 8 | GC90-4mu-DUBA | 1.72 |
| 9 | hYP7HM-DUBA | 1.53 |

9.2 Random conjugation

The single antigen solution was replaced with 20 mM NaAc-HAc buffer (pH 5.5), a concentration was adjusted to 5 ± 1 mg/mL, a desired amount of tris(2-carboxyethyl)phosphine hydrochloride (TCEP) was added at a molar ratio of 20: 1 (antibody : molar ratio) to the reaction system and mixed well, and incubated at 37°C for 3 h for reduction reaction. After the reduction reaction, the reaction mixture was centrifuged to prepare a 20 mM NaAc/Tris, 1 mM EDTA, pH 7.0 buffer solution (3,500×g, 4 cycles) using a 10 KD ultrafiltration centrifuge tube, and a concentration of the mixture was adjusted to 5±1 mg/mL to obtain a reaction solution. An appropriate amount of DMSO (~10%) was added to the above reaction solution, and an LP solution was added (5-10 mM stock solution in DMSO) (at a molar ratio of LP drug : antibody = 10: 1) to the reaction system and mixed well to obtain a mixture, and the mixture was incubated at 21-25°C for 1 h. N-acetylcysteine (NAC) was added at a molar ratio of 40: 1 (relative to the antibody) to an NAC solution and incubated at 21-25°C to terminate the reaction for 1 h. After the reduction reaction, the reaction mixture was centrifuged to prepare a 20 mM His/His-HCl, pH 6.0 buffer solution (3,500×g, 4 cycles) using a 10 KD ultrafiltration centrifuge tube, and a concentration of the mixture was adjusted to a target concentration to obtain a mixture, which was then filtered through a 0.22 μm sterile filter, and stored at ≤-20°C.

After the conjugation reaction, random conjugation products of IgG1-Dxd and GC90-4mu-Dxd were obtained. A purity of the ADC products was analyzed by size-exclusion chromatography (SEC), and a drug-to-antibody ratio (DAR) and unconjugated antibody fraction were analyzed by hydrophobic interaction chromatography (HIC).

**Example 10**

Cytotoxicity of antibody-drug conjugates on tumor cells

The cytotoxicity of the ADCs against HepG2, Hep3B, and Huh7 hepatocellular carcinoma cells was determined using the Cell Counting Kit-8 (CCK-8, Dojindo). The specific procedure was as follows: HepG2, Hep3B, and Huh7 cells were cultured in 10% FBS (Gibco) + DMEM (Corning) medium. When a cell confluence reached more than 75%, the cells were digested with trypsin (0.25% Trypsin-EDTA) and counted, and then seeded at $1.5 \times 10^4$ cells/mL, 160 μL/well (2,400 cells/well) to 96-well plates, and incubated overnight at 37°C with 5% $CO_2$. Each antibody-drug conjugate was diluted to 333.5 nM using 10% FBS+DMEM, and 40 μL/well was added to a 96-well plate containing 160 μL/well for a 5-fold dilution, resulting in a starting concentration of 66.7 nM, and 8 serial dilutions were performed, with replicate in each well, and the plate was then incubated at 37°C with 5% $CO_2$. On Day 4, the viability of tumor cells was detected using a Cell Counting

Kit-8. The specific cytotoxicity is shown in FIG. 7. With increasing drug concentration, the antibody-drug conjugate of the present invention showed good dose-dependent cytotoxicity against various hepatocellular carcinoma cells.

**Example 11**

11.1 Antitumor efficacy assay 1 of test substance

A subcutaneous xenograft model of human Hep3B hepatocellular carcinoma cells in nude mice was established.

Hep3B cells in an exponential growth phase were collected and resuspended in a 1: 1 mixture of PBS and matrix gel to adjust a cell density to $5 \times 10^7$ cells/mL. $5 \times 10^6$ Hep3B cells (0.1 mL/mouse) were subcutaneously inoculated into a right anterior dorsal region of each mouse, and tumor growth was monitored regularly. When tumors grew to an average volume greater than 100 $mm^3$, mice were randomly divided into groups for administration based on tumor size, with 6 mice each group. The day of grouping was defined as Day 0. On the day of grouping, mice were administered a single tail intravenous injection of IgG1-PBD or GC90-4mu-PBD at 1.5 mg/kg. Tumor volume was measured twice each week. A formula for tumor volume calculation is as follows:

$$\text{Tumor volume (mm}^3) = 1/2 \times (a \times b^2)$$

(where a denotes a long diameter and b denotes a short diameter).

Results: data indicated that GC90-4mu-PBD (1.5 mg/kg) exhibited significant antitumor activity (FIG. 8A).

11.2 Antitumor efficacy assay 2 of test substance

A subcutaneous xenograft model of human Hep3B hepatocellular carcinoma cells in nude mice was established.

Hep3B cells in an exponential growth phase were collected and resuspended in a 1: 1 mixture of PBS and matrix gel to adjust a cell density to $5 \times 10^7$ cells/mL. $5 \times 10^6$ Hep3B cells (0.1 mL/mouse) were subcutaneously inoculated into a right anterior dorsal region of each mouse, and tumor growth was monitored regularly. When tumors grew to an average volume greater than 100 $mm^3$, mice were randomly divided into groups for administration based on tumor size, with 6 mice each group. The day of grouping was defined as Day 0. On the day of grouping, mice were administered a single tail intravenous injection of IgG1-DUBA, hYP7HM-DUBA or GC90-4mu-DUBA at 5 or 10 mg/kg. Tumor volume was measured twice each week. A formula for tumor volume calculation is as follows:

$$\text{Tumor volume (mm}^3) = 1/2 \times (a \times b^2)$$

(where a denotes a long diameter and b denotes a short diameter).

Results: data indicated that both GC90-4mu-DUBA (5 mg/kg) and hYP7HM-DUBA (10mg/kg) exhibited significant antitumor activity (FIG. 8B).

11.3 Antitumor efficacy assay 3 of test substance

A subcutaneous xenograft model of human Hep3B hepatocellular carcinoma cells in nude mice was established.

Hep3B cells in an exponential growth phase were collected and resuspended in a mixture of PBS and matrix gel (with a ratio of 1: 1) to adjust a cell density to $5 \times 10^7$ cells/mL. $5 \times 10^6$ Hep3B cells (0.1 mL/mouse) were subcutaneously inoculated into a right anterior dorsal region of each mouse, and tumor growth was monitored regularly. When tumors grew to an average volume greater than 80-150 $mm^3$ (a main experimental group) or 300-500 $mm^3$ (a satellite group), mice were randomly divided into groups for administration based on tumor size, with 6 mice each group (1 mouse in a satellite IgG-PBD group). The second day after grouping was defined as Day 0. On Day 0, each mouse in the satellite group received a single tail intravenous injection of 1.5 mg/kg GC90-4mu-PBD or IgG1-PBD, blood and tumor samples of mice in the satellite IgG-PBD group were collected at a maximum volume 24 h after the administration. In the GC90-4mu-PBD group, blood and tumor samples were collected alternately (serum was collected at 15 min, and serum and tumor samples were collected at 7 h, D1, D2, D3, D5, and D7). On Day 0, each mouse in the main experimental group received a single tail intravenous injection of IgG1-PBD (0.6 mg/kg), GC90-4mu-PBD (0.1, 0.3, 0.6 mg/kg), or GC90-4mu-Dxd (3, 10 mg/kg), and mouse body weights and tumor sizes of the mice were measured twice each week. A formula for tumor volume calculation is as follows:

$$\text{Tumor volume (mm}^3\text{)} = 1/2 \times (a \times b^2)$$

(where denotes a long diameter and b denotes a short diameter).

**Example 12**

Functional characterization of GC90 antibody

12.1 Cross-species reactivity

The binding of GC90 to GPC3 proteins of different species was determined using ELISA. The specific procedure was as follows: each GPC3 protein (6 μg/mL) was coated onto a 96-well plate at 100 μL/well and incubated overnight at 4°C. The plate was then blocked at 37°C for 2 h with PBST (containing 0.05% Tween-20) containing 1% BSA (200 μL/well), and was then washed three times with PBST. GC90 was first serially diluted in PBST containing 1% BSA and then added sequentially to the 96-well plate (100 μL/well), with working concentrations of 30 nM and 15 nM, with a total of 8 additional points generated by 4-fold serial dilutions starting from 15 nM. The plate was incubated at 37°C for 1 h and washed three times with PBST. Then, anti-human IgG-Fc-HRP (Sigma, 1: 30,000 dilution) was added at 100 μL/well and incubated at 37°C for 1 h, the plate was then washed three times with PBST, 50 μL of TMB (SURMOPICS) was added, and the reaction was stopped with 1M $H_2SO_4$. Absorbance at 450 nm was measured with a microplate reader. The results are shown in Table 8.

Table 8. Binding of GC90 to GPC3 proteins of different species

| Antigen | Binding |
|---|---|
| Mouse GPC3 (ACRO Biosystems, GP3-M52H3) | × |
| Cyno GPC3 (ACRO Biosystems, GP3-C5225) | √ |
| Human GPC3 (ACRO Biosystems, GP3-HP2E3) | √ |
| Note: √ indicates affinity recognition, and × indicates no affinity recognition. | |

12.2 Fine mapping of the recognized epitope

The amino acid sequence of GPC3-B peptide was divided into multiple overlapping peptides, and amino acid sequences of these overlapping peptides are itemized in Table 9. The overlapping peptides were synthesized and their binding characteristics to GC90 were measured using ELISA to further precisely identify the antigenic epitopes recognized by GC90. The specific procedure was as follows: each overlapping peptide (6 μg/mL) was coated onto a 96-well plate at 100 μL/well and incubated overnight at 4°C. The plate was then blocked at 37°C for 2 h with PBST (containing 0.05% Tween-20) containing 1% BSA (200 μL/well), and was then washed three times with PBST. GC90 was serially diluted (1% BSA as a negative control) in PBST containing 1% BSA and added sequentially to the 96-well plate (100 μL/well), with working concentrations of 30 nM and 15 nM, with a total of 8 additional points generated by 4-fold serial dilutions starting from 15 nM. The plate was incubated at 37°C for 1 h and washed three times with PBST. Then, anti-human IgG-Fc-HRP (Sigma, 1: 30,000 dilution) was added at 100 μL/well and incubated at 37°C for 1 h, the plate was then washed three times with PBST, 50 μL of TMB (SURMOPICS) was added, and the reaction was stopped with 1M $H_2SO_4$. Absorbance at 450 nm was measured with a microplate reader. The results are shown in FIG. 9. $OD_{450}$ values of the measured antigen-antibody complexes illustrate the binding strength between GC90 and each peptide. It is evident that the antibody exhibits strong binding activity only with GPC3-B3 or long fragments containing GPC3-B3, demonstrating that the GPC3 antigenic epitope region is located within GPC3-B3, corresponding to at positions 485-496 of the human GPC3 protein (DKNLDEEGFESG).

Table 9. Amino acid sequences of overlapping peptides

| S/N | Amino acid sequence |
|---|---|
| GPC3-B1 | SMPKGRVLDKNL |
| GPC3-B2 | GRVLDKNLDEEG |
| GPC3-B3 | DKNLDEEGFESG |

(continued)

| S/N | Amino acid sequence |
|---|---|
| GPC3-B4 | DEEGFESGDCGD |
| GPC3-B5 | FESGDCGDDEDE |
| GPC3-B6 | DCGDDEDECIGG |
| GPC3-B7 | DEDECIGGSGDGM |
| GPC3-B8 | SMPKGRVLDKNLDEEGFESGDCGDDEDECIGGSGDGM |

Further, based on the analysis of the amino acid sequence homology between human GPC3 protein and mouse Gpc3 protein (FIG. 10), a difference in two amino acid residues was found within the GPC3-B3 region (Cyno GPC3 protein reference Genbank ID: XP_005594665.1, also including GPC3-B3). That is, amino acid residues at positions 487 (N) and 493 (F) of the human GPC3 protein of the human GPC3 protein, combined with species cross-detection results, indicate that GC90 cannot bind to the mouse Gpc3 protein due to mutations in the mouse Gpc3 protein at positions corresponding to asparagine at the position 487 and phenylalanine at the position 493 of the human GPC3 protein, further suggesting that the positions 487 and/or 493 are one of the critical binding sites of the antigenic epitope region.

FIG. 11 summarizes the antigen recognition regions of various anti-GPC3 antibodies within the GPC3 protein. It is evident that the antigenic epitope bound by the antibody of the present invention is far removed from the antigen-binding regions of other reported anti-GPC3 antibodies, recognizing a novel GPC3 antigenic epitope. The present invention identifies a novel GPC3 antigenic epitope. Antibodies binding to this epitope exhibit high affinity and endocytosis efficiency. This epitope is used to prepare immunogens, thereby providing a new strategy for the development of anti-GPC3 antibodies, reducing uncertainty in antibody generation and screening, and facilitating the development of new antibodies with improved functions.

Although the present invention has been described through one or more embodiments, it should be understood that the present invention is not limited to these embodiments. The specification is intended to cover all substitutions, modifications, and variations falling within the spirit and scope of the appended claims. All references cited herein are incorporated by reference in their entirety.

## Claims

**1.** An anti-GPC3 antibody or an antigen-binding fragment thereof, comprising a heavy chain variable region (VH) and a light chain variable region (VL); wherein

the VH comprises a heavy chain complementarity-determining region 1 (HCDR1), an HCDR2, and an HCDR3; and the HCDR1, the HCDR2, and the HCDR3 respectively comprise a sequence having at least 85%, 90%, 91%, 92%, 93%, 94%, 95%, 96%, 97%, 98%, 99%, or 100% identity to a CDR1, a CDR2, and a CDR3 of any one of the amino acid sequences shown in SEQ ID NOs: 1-2 and 6-9; or
the HCDR1, the HCDR2, and the HCDR3 respectively comprise a sequence having at most 3, 2, or 1 mutation(s) compared with the CDR1, the CDR2, and the CDR3 of any one of the amino acid sequences shown in SEQ ID NOs: 1-2 and 6-9;
the VL comprises a light chain complementarity-determining region 1 (LCDR1), an LCDR2, and an LCDR3; and the LCDR1, the LCDR2, and the LCDR3 respectively comprise a sequence having at least 85%, 90%, 91%, 92%, 93%, 94%, 95%, 96%, 97%, 98%, 99%, or 100% identity to a CDR1, a CDR2, and a CDR3 of an amino acid sequence shown in SEQ ID NO: 3; or
the LCDR1, the LCDR2, and the LCDR3 respectively comprise a sequence having at most 3, 2, or 1 mutation(s) compared with the CDR1, the CDR2, and the CDR3 of the amino acid sequence shown in SEQ ID NO: 3.

**2.** The antibody or an antigen-binding fragment thereof according to claim 1, wherein the antibody or an antigen-binding fragment thereof has the following biological functions: specific binding to an antigen shown in SEQ ID NOs: 37 or 38, and not binding to an antigen shown in SEQ ID NO: 36.

**3.** The antibody or an antigen-binding fragment thereof according to claim 1, wherein position(s) of the mutation(s) is/are selected from one or more of a position 56 (D56), a position 100 (Q100) or a position 102 (S102) of any one of the amino acid sequences shown in SEQ ID NOs: 1-2 and 6-9; and
preferably, the mutation(s) is/are selected from Q100R, S102R, D56A, D56K+Q100R, D56A+S102R or

D56K+S102R.

4. The antibody or an antigen-binding fragment thereof according to claim 3, wherein the HCDR1, HCDR2, and HCDR3 respectively comprise a sequence identical to the CDR1, the CDR2, and the CDR3 of any one of the amino acid sequences shown in SEQ ID NOs: 1-2, 6-9, and 23-28; and the LCDR1, the LCDR2, and the LCDR3 respectively comprise a sequence identical to the CDR1, the CDR2, and the CDR3 of the amino acid sequence shown in SEQ ID NO: 3.

5. The antibody or an antigen-binding fragment thereof according to any one of claims 1-4, wherein the CDR1, the CDR2, and the CDR3 are defined according to IMGT, Kabat, Chothia, AbM, or Contact.

6. The antibody or an antigen-binding fragment thereof according to claim 5, wherein the VH comprises a sequence having at least 85%, 90%, 91%, 92%, 93%, 94%, 95%, 96%, 97%, 98%, 99%, or 100% identity to any one of the amino acid sequences shown in SEQ ID NOs: 1-2, 6-9, and 23-28; and/or
the VL comprises a sequence having at least 85%, 90%, 91%, 92%, 93%, 94%, 95%, 96%, 97%, 98%, 99%, or 100% identity to the amino acid sequence shown in SEQ ID NO: 3.

7. The antibody or an antigen-binding fragment thereof according to claim 6, wherein the VH comprises a sequence identical to any one of the amino acid sequences shown in SEQ ID NOs: 1-2, 6-9, and 23-28; and the VL comprises a sequence identical to the amino acid sequence shown in SEQ ID NO: 3.

8. The antibody or an antigen-binding fragment thereof according to any one of claims 1-7, further comprising a heavy chain constant region (CH) and a light chain constant region (CL); preferably, the CH comprises a sequence identical to any one of the amino acid sequences shown in SEQ ID NOs: 4, 19, or 20; and the CL comprises a sequence identical to an amino acid sequence shown in SEQ ID NO: 5.

9. The antibody or an antigen-binding fragment thereof according to claim 8, wherein the heavy chain (H) comprises a sequence having at least 85%, 90%, 91%, 92%, 93%, 94%, 95%, 96%, 97%, 98%, 99%, or 100% identity to any one of the amino acid sequences shown in SEQ ID NOs: 11-18 and 29-34; and/or the light chain (L) comprises a sequence having at least 85%, 90%, 91%, 92%, 93%, 94%, 95%, 96%, 97%, 98%, 99%, or 100% identity to an amino acid sequence shown in SEQ ID NO: 10.

10. The antibody or an antigen-binding fragment thereof according to claim 9, wherein the heavy chain (H) comprises a sequence identical to any one of the amino acid sequences shown in SEQ ID NOs: 11-18 and 29-34; and the light chain (L) comprises a sequence identical to the amino acid sequence shown in SEQ ID NO: 10.

11. An anti-GPC3 antibody or an antigen-binding fragment thereof, wherein the antibody or an antigen-binding fragment thereof binds to an antigen shown in SEQ ID NOs: 37 or 38, and does not bind to an antigen shown in SEQ ID NO: 36; or

   the antibody or an antigen-binding fragment thereof competes with a reference antibody for binding to the same epitope of a GPC3 protein, wherein the reference antibody comprises a heavy chain shown in SEQ ID NO: 11 and a light chain shown in SEQ ID NO: 10; and
   preferably, the anti-GPC3 antibody or an antigen-binding fragment thereof inhibits the binding of the reference antibody to the GPC3 protein by at least 50%, 60%, 70%, 80%, 90%, 95%, or 99%.

12. A GPC3 antigen epitope peptide, wherein the GPC3 antigen epitope peptide is formed by at least 7 consecutive amino acid residues from residues 485-496 of a human GPC3 protein, and an amino acid sequence of the human GPC3 protein is shown in SEQ ID NO: 35; and the GPC3 antigen epitope peptide comprises at least one of asparagine at a position 487 and phenylalanine at a position 493, and the GPC3 antigen epitope peptide has one or more of the following biological functions:

   (1) specifically binding to an anti-GPC3 antibody;
   (2) inducing an immune response against GPC3 (e.g., a humoral immune response) in a subject;
   (3) inducing the production of an anti-GPC3 antibody in a subject; and
   (4) preventing and/or treating GPC3-related diseases in a subject.

13. The GPC3 antigen epitope peptide according to claim 12, wherein the GPC3 antigen epitope peptide is formed by an amino acid sequence shown in SEQ ID NO: 38.

**14.** A recombinant antigen, comprising the GPC3 antigen epitope peptide according to claim 12 or 13 and a carrier protein.

**15.** A chimeric antigen receptor, comprising the anti-GPC3 antibody or an antigen-binding fragment thereof according to any one of claims 1-11.

**16.** A polynucleotide encoding the anti-GPC3 antibody or an antigen-binding fragment thereof according to any one of claims 1-11, the GPC3 antigen epitope peptide according to any one of claims 12-13, or the recombinant antigen according to claim 14.

**17.** An expression vector, comprising the polynucleotide according to claim 16.

**18.** A host cell, comprising the polynucleotide according to claim 16 or the expression vector according to claim 17.

**19.** An antibody-drug conjugate, comprising the anti-GPC3 antibody or an antigen-binding fragment thereof according to any one of claims 1-11, and a drug or a toxin.

**20.** The antibody-drug conjugate according to claim 19, wherein the drug or the toxin is selected from one or more of SN-38, MMAE, a PBD dimer, DX-8951 (DXd), or DUBA.

**21.** A bispecific or a multispecific antibody molecule, comprising the anti-GPC3 antibody or an antigen-binding fragment thereof according to any one of claims 1-11.

**22.** A pharmaceutical composition, comprising the anti-GPC3 antibody or an antigen-binding fragment thereof according to any one of claims 1-11, or the GPC3 antigen epitope peptide according to any one of claims 12-13, or the recombinant antigen according to claim 14, or the chimeric antigen receptor according to claim 15, or the polynucleotide according to claim 16, or the expression vector according to claim 17, or the host cell according to claim 18, or the antibody-drug conjugate according to any one of claims 19-20, or the bispecific or the multispecific antibody molecule according to claim 21; and at least one pharmaceutically acceptable carrier.

**23.** A kit, comprising the anti-GPC3 antibody or an antigen-binding fragment thereof according to any one of claims 1-11, or the GPC3 antigen epitope peptide according to any one of claims 12-13, or the recombinant antigen according to claim 14, or the antibody-drug conjugate according to any one of claims 19-20, or the bispecific or the multispecific antibody molecule according to claim 21.

**24.** Use of the anti-GPC3 antibody or an antigen-binding fragment thereof according to any one of claims 1-11, the GPC3 antigen epitope peptide according to any one of claims 12-13, the recombinant antigen according to claim 14, the chimeric antigen receptor according to claim 15, the polynucleotide according to claim 16, the expression vector according to claim 17, the host cell according to claim 18, the antibody-drug conjugate according to any one of claims 19-20, the bispecific or the multispecific antibody molecule according to claim 21, or the kit according to claim 23 in the preparation of a medicament for treating or preventing cancer; and preferably the cancer is liver cancer.

**25.** Use of the GPC3 antigen epitope peptide according to any one of claims 12-13 or the recombinant antigen according to claim 14 in any one of the following:

(1) preparing an anti-GPC3 antibody or an antigen-binding fragment thereof;
(2) preparing a product for treating and/or preventing and/or diagnosing GPC3-related diseases in a subject;
(3) preparing a product for detecting an anti-GPC3 antibody or an antigen-binding fragment thereof;
(4) detecting an anti-GPC3 antibody or an antigen-binding fragment thereof; and
(5) screening for an anti-GPC3 antibody or an antigen-binding fragment thereof.

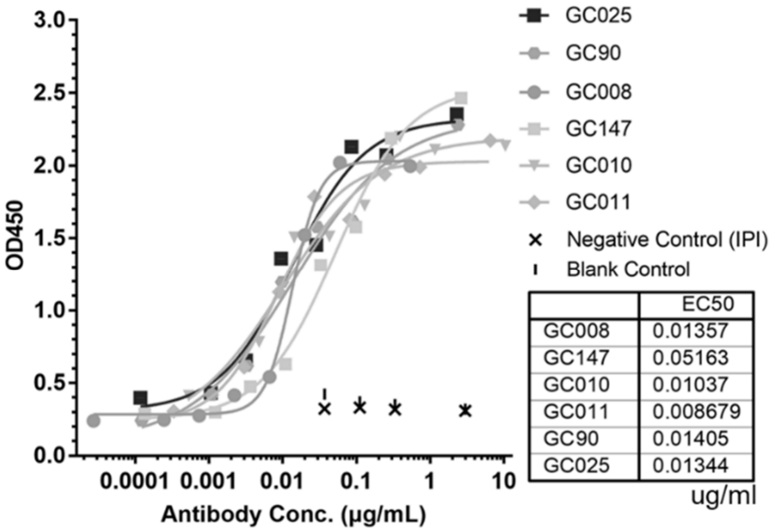


FIG. 1A

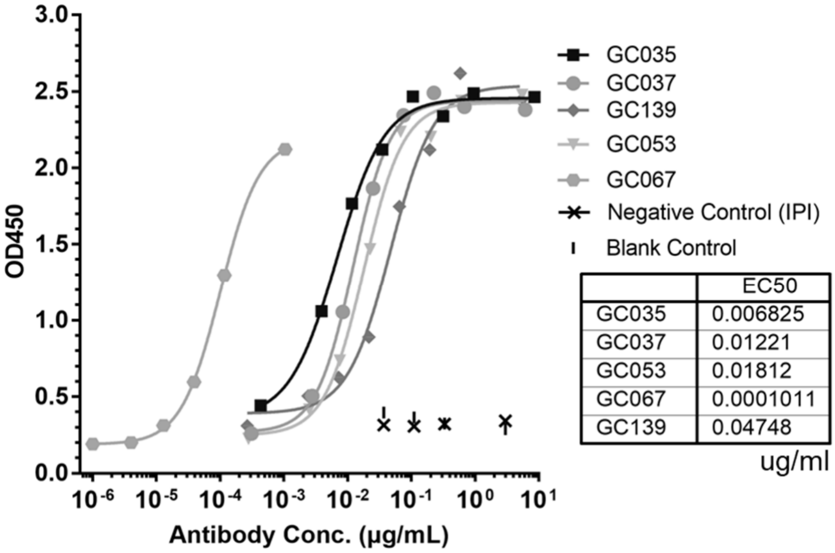


FIG. 1B

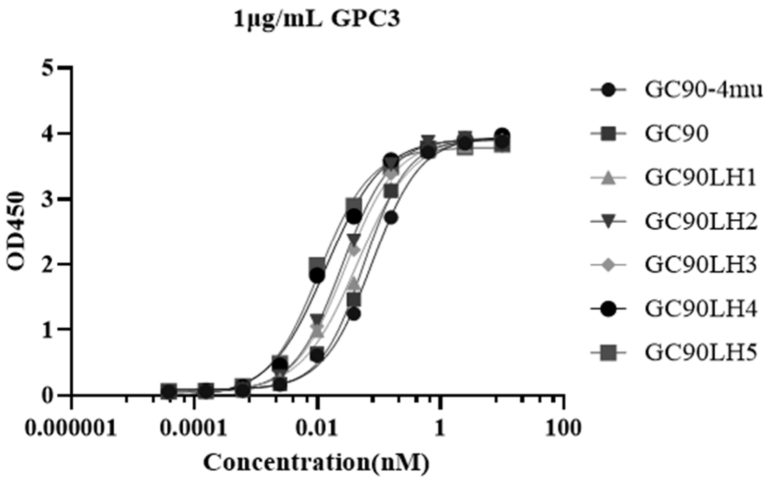


FIG. 2

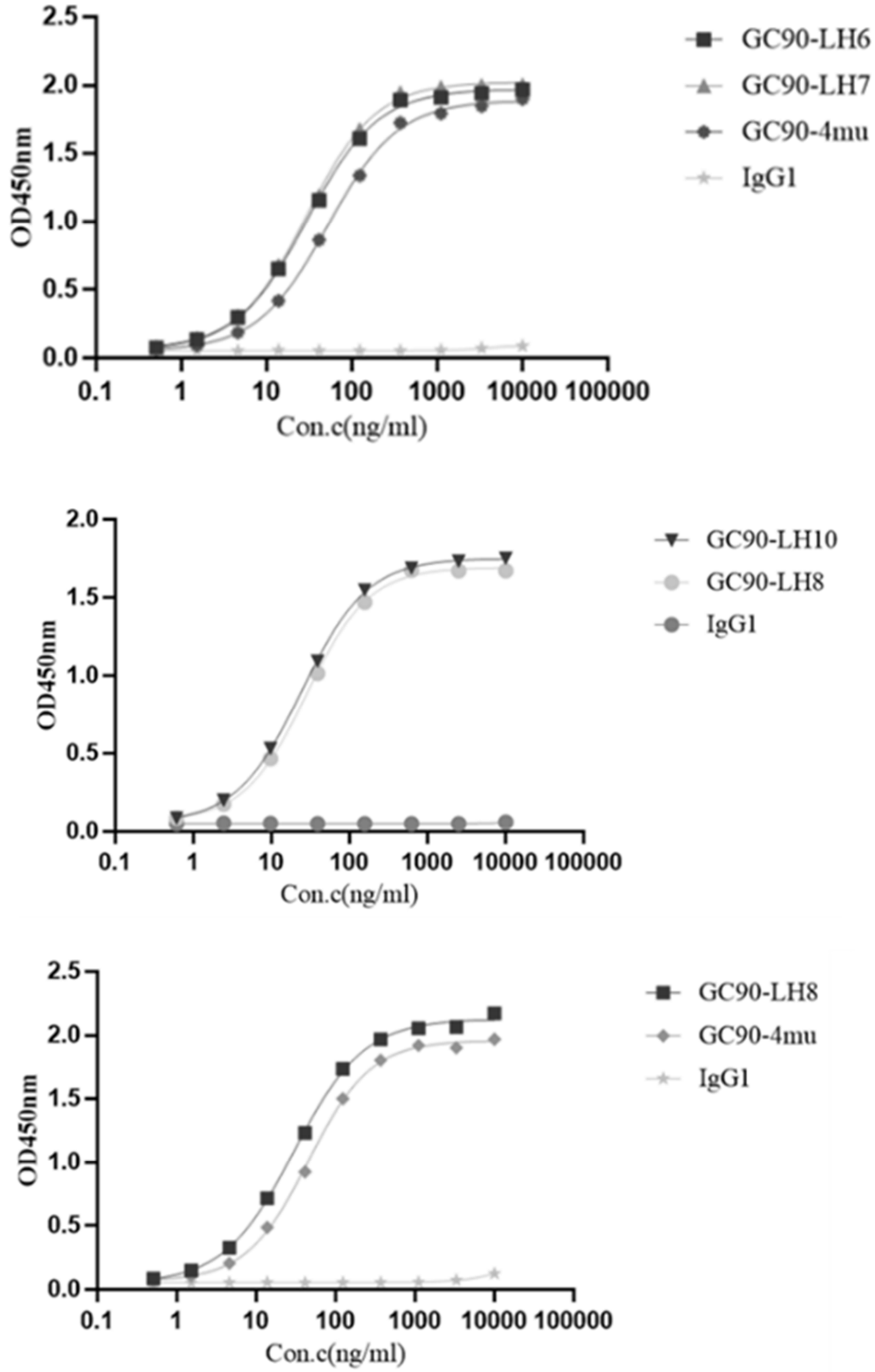

GC90-LH6
GC90-LH7
GC90-4mu
IgG1
OD450nm
Con.c(ng/ml)
GC90-LH10
GC90-LH8
IgG1
OD450nm
Con.c(ng/ml)
GC90-LH8
GC90-4mu
IgG1
OD450nm
Con.c(ng/ml)


FIG. 3

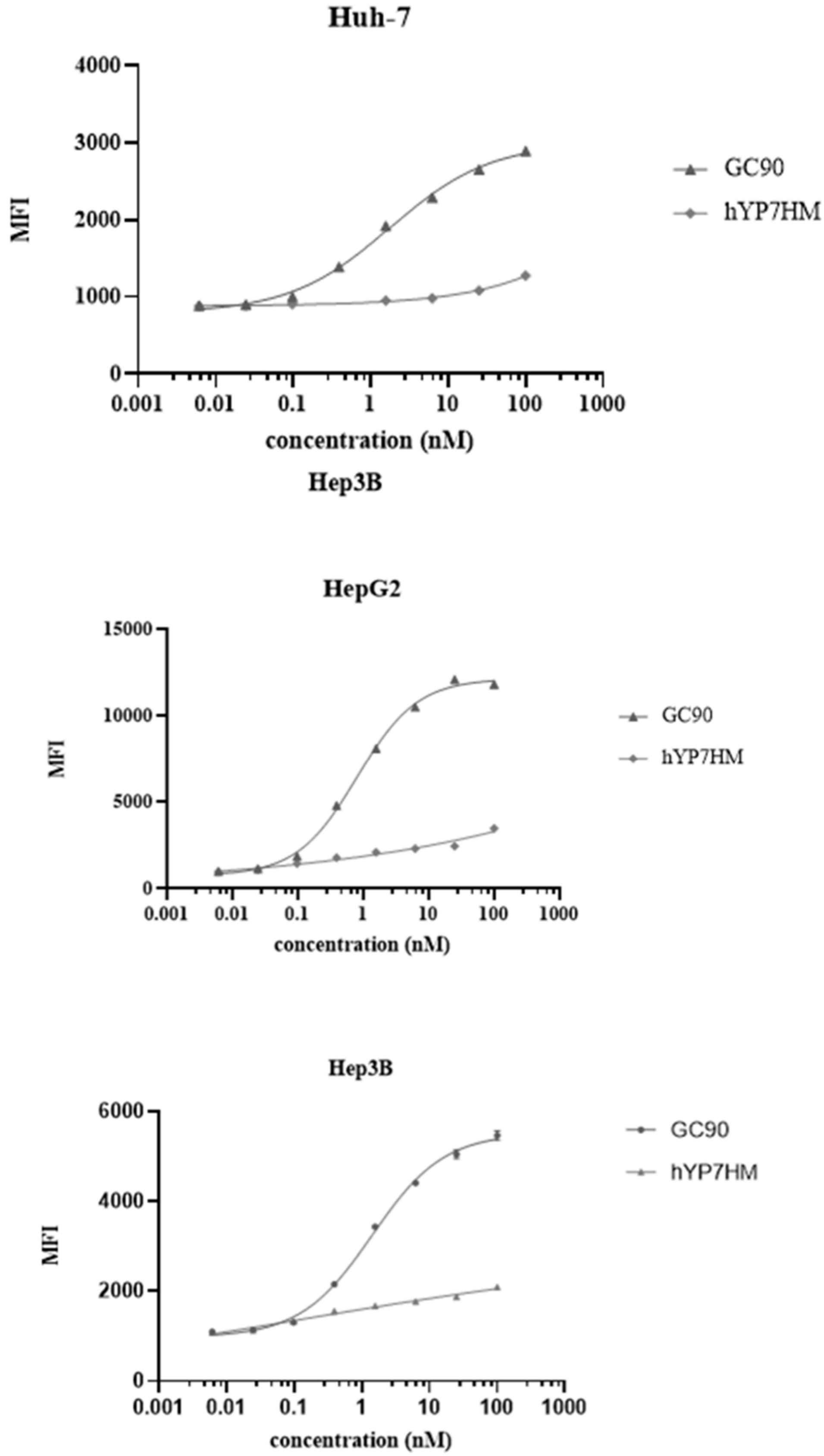
Huh-7
MFI
4000
3000
2000
1000
0
0.001
0.01
0.1
1
10
100
1000
concentration (nM)
GC90
hYP7HM
Hep3B
HepG2
MFI
15000
10000
5000
0
0.001
0.01
0.1
1
10
100
1000
concentration (nM)
GC90
hYP7HM
Hep3B
MFI
6000
4000
2000
0
0.001
0.01
0.1
1
10
100
1000
concentration (nM)
GC90
hYP7HM

FIG. 4

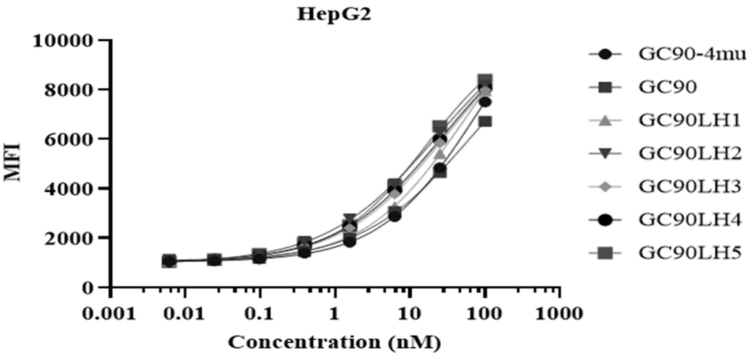


FIG. 5A

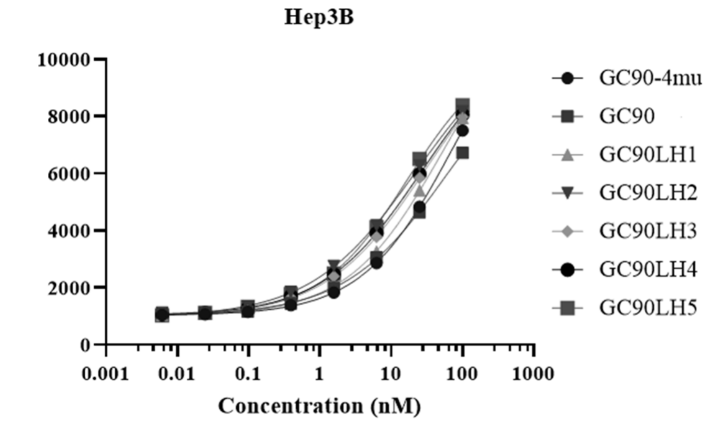


FIG. 5B

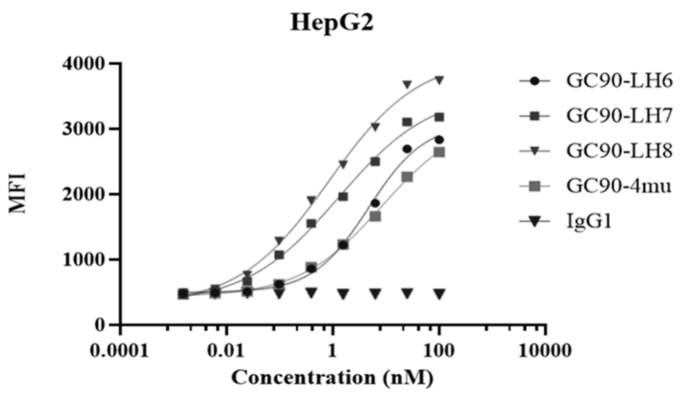


FIG. 5C

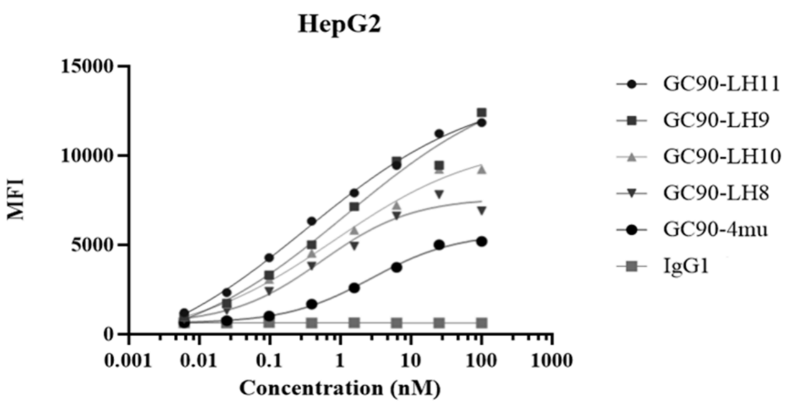


FIG. 5D

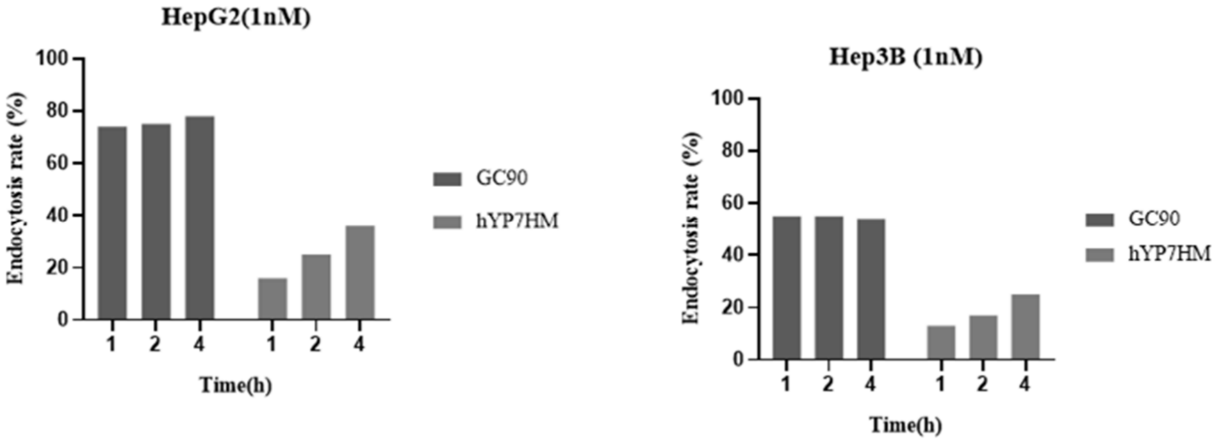
HepG2(1nM)
Endocytosis rate (%)
100
80
60
40
20
0
1
2
4
1
2
4
Time(h)
GC90
hYP7HM
Hep3B (1nM)
Endocytosis rate (%)
100
80
60
40
20
0
1
2
4
1
2
4
Time(h)
GC90
hYP7HM

FIG. 6

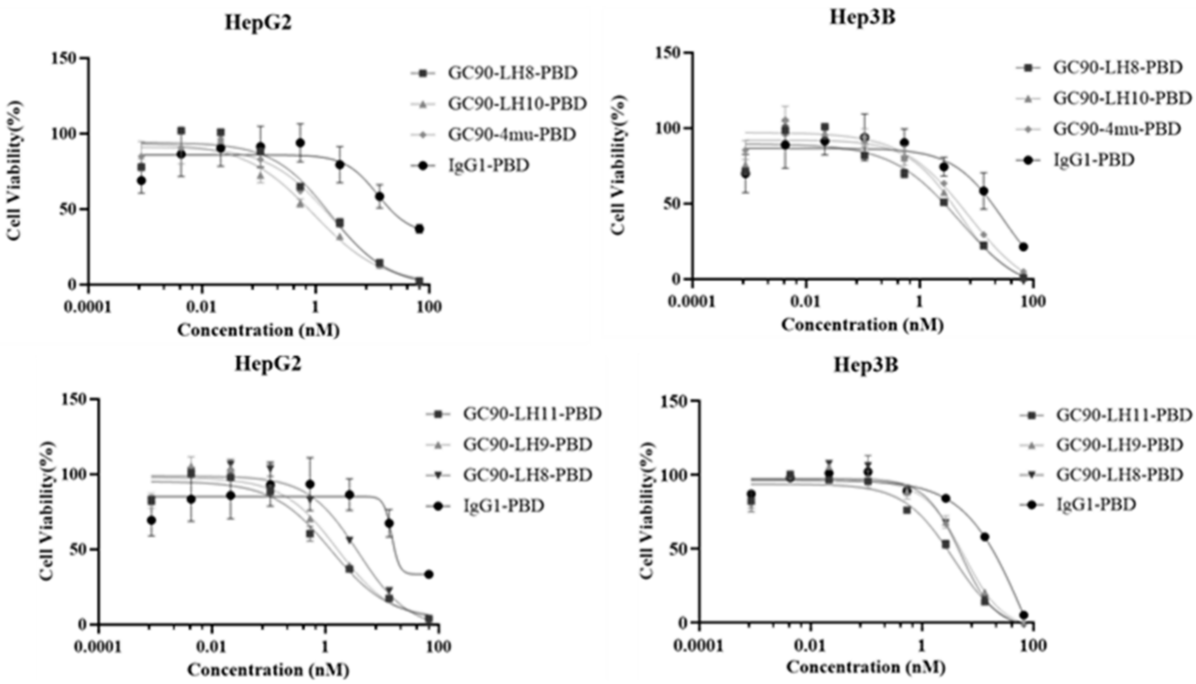
HepG2
Cell Viability(%)
Concentration (nM)
GC90-LH8-PBD
GC90-LH10-PBD
GC90-4mu-PBD
IgG1-PBD
Hep3B
Cell Viability(%)
Concentration (nM)
GC90-LH8-PBD
GC90-LH10-PBD
GC90-4mu-PBD
IgG1-PBD
HepG2
Cell Viability(%)
Concentration (nM)
GC90-LH11-PBD
GC90-LH9-PBD
GC90-LH8-PBD
IgG1-PBD
Hep3B
Cell Viability(%)
Concentration (nM)
GC90-LH11-PBD
GC90-LH9-PBD
GC90-LH8-PBD
IgG1-PBD
150
100
50
0
0.0001
0.01
1
100

FIG. 7

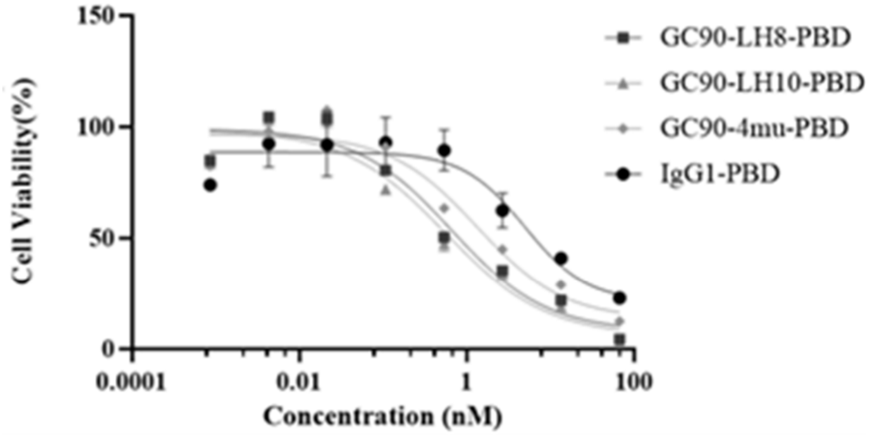


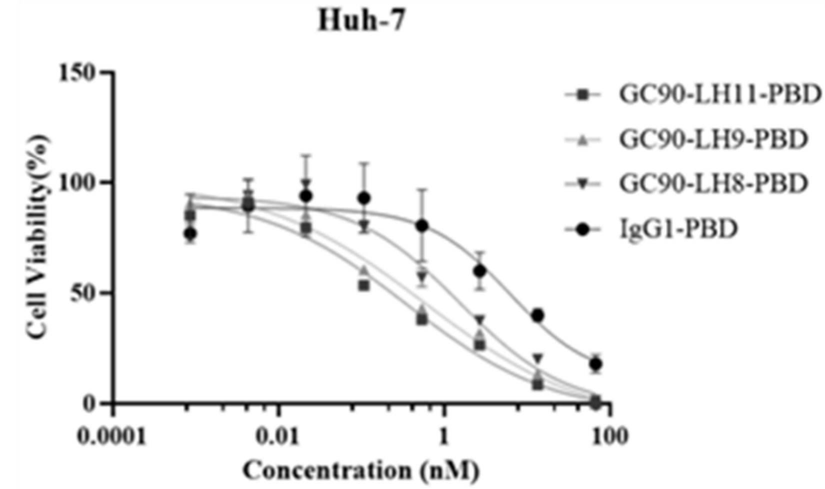


FIG. 7 (continued)

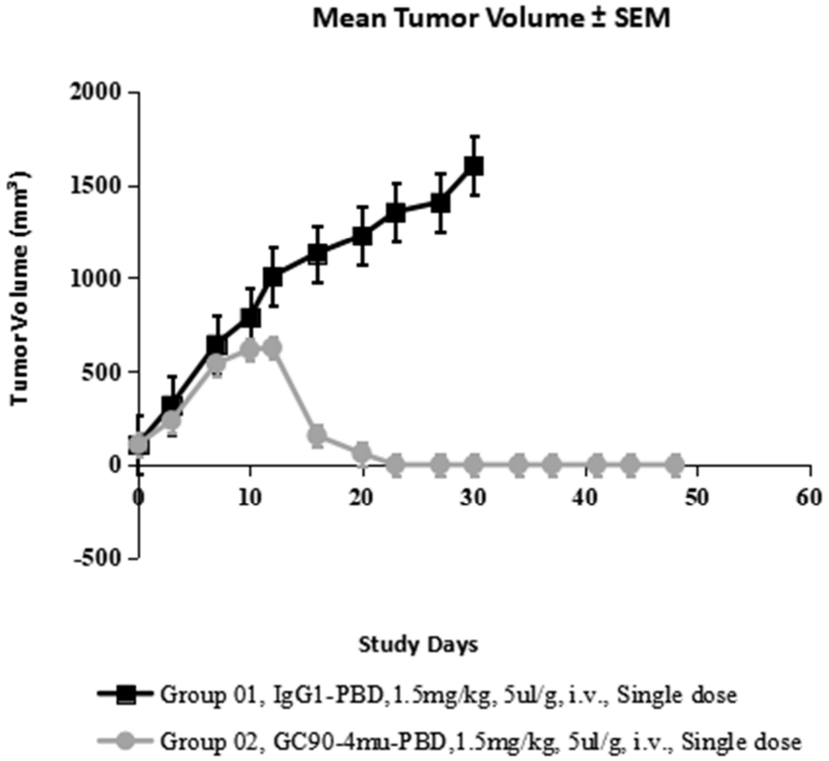


FIG. 8A

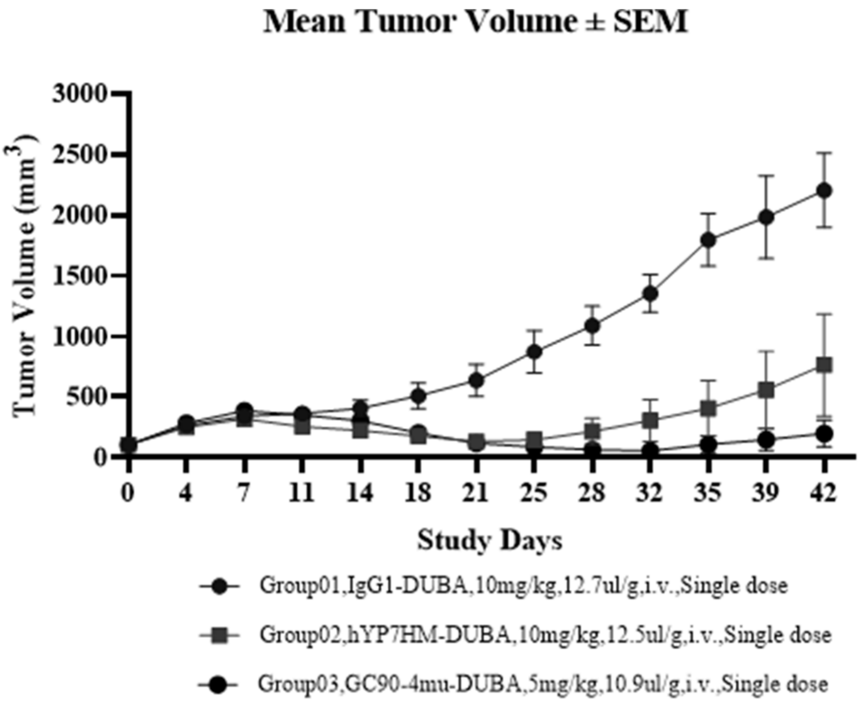


FIG. 8B

| | |
|---|---|
| GPC3-B1 | **SMPKGRVLDKNL**DEEGFESGDCGDDEDECIGGSGDGM |
| GPC3-B2 | SMPK**GRVLDKNLDEEG**FESGDCGDDEDECIGGSGDGM |
| GPC3-B3 | SMPKGRVL**DKNLDEEGFESG**DCGDDEDECIGGSGDGM |
| GPC3-B4 | SMPKGRVLDKNL**DEEGFESGDCGD**DEDECIGGSGDGM |
| GPC3-B5 | SMPKGRVLDKNLDEEG**FESGDCGDDEDE**CIGGSGDGM |
| GPC3-B6 | SMPKGRVLDKNLDEEGFESG**DCGDDEDECIGG**SGDGM |
| GPC3-B7 | SMPKGRVLDKNLDEEGFESGDCGD**DEDECIGGSGDGM** |
| GPC3-B8 | SMPKGRVLDKNLDEEGFESGDCGDDEDECIGGSGDGM |

| | GPC3-B1 | GPC3-B2 | GPC3-B3 | GPC3-B4 | GPC3-B5 | GPC3-B6 | GPC3-B7 | GPC3-B8 |
|---|---|---|---|---|---|---|---|---|
| GC90 | 0.072 | 0.07 | 3.908 | 0.071 | 0.072 | 0.075 | 0.067 | 4 |
| | 0.072 | 0.074 | 3.905 | 0.063 | 0.061 | 0.073 | 0.064 | 3.926 |
| NC | 0.062 | 0.056 | 0.068 | / | / | 0.056 | / | 0.054 |

FIG. 9

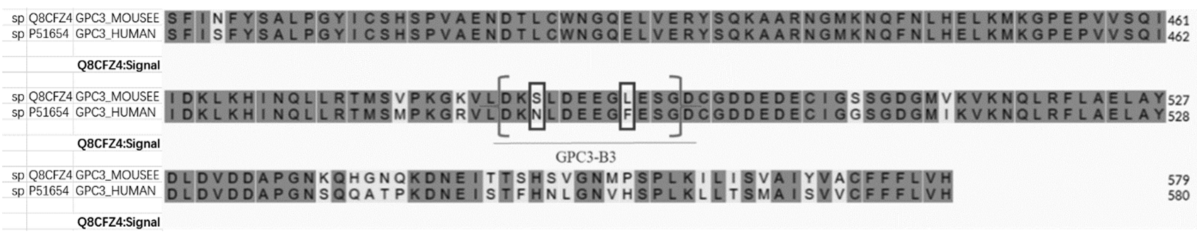


FIG. 10

>GPC3
MAGTVRTACLVVAMLLSLDFPGQAQPPPPPPDATCHQVRSFFQRLQPGLKWVPETPVPGSDLQVCLPKGPTCCSRKMEEKYQLTARLNMEQLLQSASMELKFLIIQNAAVFQEA
FEIVVRHAKNYTNAMFKNNYPSLTPQAFEFVGEFFTDVSLYILGSDINVDDMVNELFDSLFPVIYTQLMNPGLPDSALDINECLRGARRDLKVFGNFPKLIMTQVSKSLQVTRIFLQ
ALNLGIEVINTTDHLKFSKDCGRMLTRMWYCSYCQGLMMVKPCGGYCNVVMQGCMAGVVEIDKYWREYILSLEELVNGMYRIYDMENVLLGLFSTIHDSIQYVQKNAGKLTT
TIGKLCAHSQQRQYRFAYYPEDLFIDKKVLKVAHVEHEETLSSRRRELIQKLKSFISFYSALPGYICSHSPVAENDTLCWNGQELVERYSQKAARNGMKNQFNLHELKMKGPEPV
VSQIIDKLKHINQLLRTMSMPKGRVLDKNLDEEGFESGDCGDDEDECIGGS**GDGMIKVKNQLRFLAELAYDLD**VDDAPGNSQQATPKDNEISTFHNLRNVHSPLKLLTSMAISV
VCFFFLVH

GC90 recognition region

hYP7HM and GC33 recognition regions

FIG. 11

**INTERNATIONAL SEARCH REPORT**

International application No. **PCT/CN2024/099094**

**A. CLASSIFICATION OF SUBJECT MATTER**

C07K16/30(2006.01)i; C07K16/18(2006.01)i; C12N15/13(2006.01)i; A61P35/00(2006.01)i; A61K39/00(2006.01)i; A61K39/395(2006.01)i; A61K47/68(2017.01)i

According to International Patent Classification (IPC) or to both national classification and IPC

**B. FIELDS SEARCHED**

Minimum documentation searched (classification system followed by classification symbols)

IPC:C07K C12N A61P A61K

Documentation searched other than minimum documentation to the extent that such documents are included in the fields searched

Electronic data base consulted during the international search (name of data base and, where practicable, search terms used)

CNABS; CNTXT; CJFD; DWPI; ENTXTC; ENTXT; CNKI; 万方, WANFANG; ISI Web of Science; GenBank; 中国专利生物序列检索系统, China Patents Biological Sequence Search System; STN: 信立泰, 牟宗春, 磷脂酰肌醇蛋白聚糖3, 抗体, 单抗, 单克隆抗体, 肿瘤, 癌, GPC3, GPC-3, Glypican-3, antibody, mAb, monoclonal antibody, tumor, cancer, 序列1-2, 6-18, 23-34, 37, 38, 序列1-2, 6-9的CDR1-3, 序列3的CDR1-3, Q100R, S102R, D56A, D56K+Q100R, D56A+S102R, D56K+S102R

**C. DOCUMENTS CONSIDERED TO BE RELEVANT**

| Category* | Citation of document, with indication, where appropriate, of the relevant passages | Relevant to claim No. |
|---|---|---|
| X | CN 115298216 A (SHANGHAI HANSOH BIOMEDICAL CO., LTD. et al.) 04 November 2022 (2022-11-04)<br>claims 1-22 | 11, 15-24 |
| A | CN 109988240 A (ANYUAN BIOTECHNOLOGY (SHANGHAI) CO., LTD. et al.) 09 July 2019 (2019-07-09)<br>claims 1-19 | 1-25 |
| A | CN 112175085 A (XIAMEN UNIVERSITY et al.) 05 January 2021 (2021-01-05)<br>claims 1-15 | 1-25 |
| A | WO 2022171100 A1 (JIANGSU SIMCERE PHARMACEUTICAL CO., LTD.) 18 August 2022 (2022-08-18)<br>claims 1-29 | 1-25 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 September 2024** | **23 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/099094**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/099094**

| Patent document cited in search report | | | Publication date (day/month/year) | Patent family member(s) | | | Publication date (day/month/year) |
|---|---|---|---|---|---|---|---|
| CN | 115298216 | A | 04 November 2022 | TW | 202144433 | A | 01 December 2021 |
| | | | | WO | 2021213245 | A1 | 28 October 2021 |
| CN | 109988240 | A | 09 July 2019 | | None | | |
| CN | 112175085 | A | 05 January 2021 | | None | | |
| WO | 2022171100 | A1 | 18 August 2022 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003000883 A
- WO 2004022739 A
- WO 03100429 A
- WO 2004018667 A
- CN 1842540 B
- CN 10452033 B
- CN 115850492 A


### Non-patent literature cited in the description


- Remington: The Science and Practice of Pharmacy,. Lippincott Williams & Wilkins, 2003
- *CHEMICAL ABSTRACTS*, 1279680-68-0
- *CHEMICAL ABSTRACTS*, 646502-53-6
- *CHEMICAL ABSTRACTS*, 1595275-62-9
- *CHEMICAL ABSTRACTS*, 1599440-13-7
- *CHEMICAL ABSTRACTS*, 1345681-58-4
- *CHEMICAL ABSTRACTS*, 86639-52-3
- *CHEMICAL ABSTRACTS*, 474645-27-7
- *CHEMICAL ABSTRACTS*, 1595275-71-0
- *CHEMICAL ABSTRACTS*, 171335-80-1
- **MUNSON et al.** *Anal. Biochem.*, 1980, vol. 107, 220
- **WARD et al.** *Nature*, 1989, vol. 341, 544-546
- **BIRD et al.** *Science*, 1988, vol. 242, 423-426
- **HUSTON et al.** *Proc. Natl. Acad. Sci.*, 1988, vol. 85, 5879-5883
- Epitope Mapping Protocols in Methods in Molecular Biology. 1996, vol. 66